# EUROPÄISCHE PATENTSCHRIFT

(11) **EP 2 374 445 B1**
(45) Veröffentlichungstag und Bekanntmachung des Hinweises auf die Patenterteilung: **29.07.2015**
(21) Anmeldenummer: 11162129.8
(22) Anmeldetag: 12.04.2011
(51) Int. Cl.: A61K 6/083

(54) **Dualhärtende, mehrkomponentige dentale Zusammensetzung**
Dual hardening multi-component dental composition
Composition dentaire multicomposant à durcissement double

(30) Priorität: 12.04.2010 DE 102010003884
(43) Veröffentlichungstag der Anmeldung: 12.10.2011
(73) Patentinhaber: VOCO GmbH, 27472 Cuxhaven (DE)
(72) Erfinder: Maletz, Reinhard, 27472 Cuxhaven (DE); Krumme, Wigand, 27476 Cuxhaven (DE); Plaumann, Manfred Thomas, 27472 Cuxhaven (DE)
(74) Vertreter: Eisenführ Speiser

(56) Entgegenhaltungen:
- EP-A1- 2 072 028
- DE-A1- 1 795 395
- DE-C1- 4 233 886
- US-A- 4 369 296
- US-A- 4 490 497

## Beschreibung

Die vorliegende Erfindung betrifft eine dualhärtende, mehrkomponentige dentale Zusammensetzung enthaltend ein oder mehrere bestimmte Additive (Molekulargewichtsregler), ein entsprechendes Verfahren zur Herstellung einer solchen Zusammensetzung sowie die Verwendung bestimmter Molekulargewichtsregler zur Herstellung einer dualhärtenden, mehrkomponentigen dentalen Zusammensetzung.

Lichthärtbare Kompositmaterialien sind die am häufigsten eingesetzten zahnärztlichen Werkstoffe. Moderne dentale Komposite sind meist eine Kombination aus einer organischen Kunststoffmatrix mit anorganischen Füllstoffen, wobei die anorganische Phase mit der organischen Phase verbunden ist. Die noch nicht vernetzte Zusammensetzung enthält somit freie Monomere bzw. Monomermischungen, anorganische Füllstoffe bzw. Füllstoffmischungen, Initiatoren und zumeist Farbstoffe.

Die Aushärtung lichthärtbarer dentaler Kompositmaterialien erfolgt durch photoinitiierte Polymerisation. Hierbei werden, nach photochemischer Aktivierung eines Initiatorsystems, Kettenreaktionen eingeleitet, wobei es über eine dreidimensionale Vernetzung zu einer Überführung der flüssigen Monomeren zu einem festen Polymerisat kommt.

Die Versorgung von präparierten Zähnen mit Provisorien beispielsweise in der Kronen- und Brückenprothetik ist für Patienten von wesentlicher Bedeutung, da durch sie eine korrekte Ästhetik, Phonetik und Okklusion gewährleistet wird. Sie schützt darüberhinaus sowohl die Zahnhartsubstanz vor Karies und Frakturen als auch die Pulpa vor Trauma oder Verletzungen. Sie bewahrt ferner die Dentinwunde des beschliffenen Zahns vor thermische, chemische, mechanische und bakteriologische Reize, die innerhalb kürzester Zeit zu entzündlichen Vorgängen in der Pulpa führen können.

Bei der Herstellung von Provisorien können unterschiedliche Verfahren zur Anwendung kommen:
Bei der direkten Methode wird beispielsweise vor der Präparation mit Alginat oder einer Silikonmasse ein Situationsabdruck genommen. Nach der Präparation wird die Kunststoffzusammensetzung in den Abdruck eingebracht und wieder in den Mund des Patienten reponiert.

Alternativ können beispielsweise auch bereits konfektionierte Kunststoffhülsen verwendet werden, wobei das Provisorium ohne vorherigen Situationsabdruck erstellt wird. Die Hülsen werden individuell im Mund mit Schere und Fräse angepasst, mit einem selbstpolymerisierenden Kunststoff gefüllt und dann auf den präparierten Zahn aufgesetzt.

Materialien für die Herstellung provisorischer Kronen und Brücken müssen während ihrer Verweildauer im Mund das Funktionieren der Provisorien sicherstellen. Hierzu müssen die Materialien eine Vielzahl an Anforderungen erfüllen.

So sollten die Materialien während ihres Einsatzes keine toxischen Substanzen freisetzen. Die Abgabe schädlicher Stoffe in den Mundraum könnte durch Diffusion, Abrieb, Abbau sowie durch zahnärztliche Tätigkeiten wie Schleifen erfolgen. Insbesondere ist eine Freisetzung von Kunststoffmonomeren zu verhindern, die allergenes Potential aufweisen und in Form von nicht umgesetzten Restmonomeren durch Diffusion in den Mundraum entweichen könnten.

Weiterhin sollte das Material gute mechanische Eigenschaften, besonders bezüglich Härte, Festigkeit, Elastizitätsmodul und Abrasionsverhalten aufweisen, um, zusammen mit einer optimalen Anpassung an die okklusalen Verhältnisse, eine störungsfreie Funktion während der Liegezeit sicherzustellen.

Die Oberfläche des Provisoriums sollte glatt und hochglanzpolierbar sein. Die Glätte des Materials dient dazu, eine Anlagerung von Plaque zu verhindern, die Hochglanzpolierbarkeit wird angestrebt, um ästhetische Belange zu bedienen. Eine raue Oberfläche könnte zudem zu einer Reizung der Zunge führen und Mißempfindungen auslösen.

Das Provisorium sollte darüberhinaus, zusammen mit dem Befestigungsmaterial, eine hohe Randdichtigkeit vorweisen, um ein Eindringen von Bakterien und Substanzen zu verhindern.

Des Weiteren sollte das Material während der intraoralen Phase der Polymerisation keine thermischen Schäden verursachen. Durch die während der Vernetzung freigesetzte Wärmeenergie sollten keine Schmerzen im Mund des Patienten hervorgerufen werden. Gleichzeitig sollte das Material eine vollständige und dichte Abdeckung der beschliffenen Zahnfläche bewerkstelligen und so den Zahn vor thermischen und mechanischen Reizungen schützen.

Die heutzutage gebräuchlichen Kronen- und Brückenmaterialien sind kunststoffbasierend und in ihrem chemischen Aufbau recht unterschiedlich. Sie enthalten in der Regel Methacrylate, Acrylate sowie Katalysatoren, Inhibitoren, Vernetzer, Weichmacher, Beschleuniger, UV-Absorber, Füllstoffe, etc.. Die Aushärtung dieser Produkte erfolgt entweder chemisch durch Autopolymerisation nach Mischen zweier Pasten, der sogenannten Basispaste mit der sogenannten Katalysatorpaste in dem statischen Mischrohr einer Doppelkammerkartusche oder dualhärtend, d.h. sowohl chemisch als auch mittels Strahlung einer geeigneten Wellenlänge.

Entscheidender Nachteil einer autopolymerisierbaren Kunststoffzusammensetzung ist die Wärmeentwicklung bei der exothermen Polymerisation, die eine Gefahr für die Pulpa darstellt. Die freigesetzte Wärmemenge ist abhängig von der Dosierung des eingesetzten Katalysatorsystems, von der formgebenden Masse (Abformstoff) und von der Verarbeitungsmenge. In der Literatur wurden von verschiedenen Arbeitsgruppen unterschiedliche Temperaturspitzen für provisorische Kronen- und Brückenmaterialien gemessen, die je nach Versuchsanordnung und chemischer Zusammensetzung zwischen 25 und 94°C differieren. Neben der maximalen Temperatur des Polymerisationsverlaufs ist dabei auch die Form der Temperaturentwicklung von Bedeutung. So kann beispielsweise die Temperaturentwicklung der Aushärtung einer Zusammensetzung eines provisorischen Kronen- und Brückenmaterials in einem Temperatur-Zeit-Diagramm zu Beginn der Vernetzung relativ flach verlaufen, um dann, um den Zeitpunkt des Temperaturmaximums, kurz und stark anzusteigen und viel Wärme innerhalb kürzester Zeit abzugeben. Andererseits kann eine solche Kurve auch eine solche Form annehmen, dass eine relativ lang andauernde, allerdings schwächere Wärmeentwicklung stattfindet.

Selbsthärtende provisorische Kronen- und Brückenmaterialien werden üblicherweise zunächst in die Abformung appliziert. Hierbei wird die zumeist zweikomponentige Zusammensetzung durch ein statisches Mischrohr einer Doppelkammerkartusche gedrückt. Die Komponenten werden bei diesem Vorgang miteinander gemischt und somit zur Reaktion gebracht. Die mit dem gemischten Material gefüllte Abformung wird dann in den Mund eingebracht und auf den präparierten Stumpf gedrückt. Nachdem das radikalisch härtbare Kunststoffmaterial in der Abformung seine elastische Phase durchläuft, wird es nach ca. 1 Minute vom Stumpf entfernt und aus dem Mund genommen, wo es extraoral zu Ende härtet. Hier erreicht die Abbindung auch ihre Maximaltemperatur.

Bereits während der elastischen Phase, wo noch nicht das Temperaturmaximum erreicht ist, wird sehr schnell ein Zustand der dentalen Zusammensetzung erreicht, bei dem sie zu gelieren beginnt und die äußere Form nicht mehr grundsätzlich ändert. Die Viskosität der Zusammensetzung ist nun so hoch, dass es für die noch nicht abreagierten ungesättigten Gruppen immer schwerer wird, eine hinreichende Mobilität aufzunehmen, um Umsetzungspartner zu treffen. In der Regel bleiben freie Monomere im Netzwerk zurück, die später an die Umgebung im Mund abgegeben werden können.

Ein weiterer Nachteil ist die während der Vernetzung auftretende Änderung der Dichte im Material, die im ungünstigen Fall zu einer Änderung der äußeren Form führen kann. Wenn das Kronen- und Brückenmaterial jedoch nicht formstabil aushärtet, so kann es später zu Passungenauigkeiten führen. Die Krone deckt dann den Stumpf nicht mehr vollständig ab. An diesen Stellen könnten später Mikroorganismen einwandern und den Zahnstumpf von innen her angreifen. Diese Dichteänderung wird als Schrumpf oder Volumenschwindung der Reaktionsharzmasse bezeichnet. Sie hängt in erster Linie von der Anzahl der abreagierten funktionellen Gruppen ab. Der Schrumpf tritt sowohl im flüssigen Zustand, also ganz zu Beginn der Polymerisation als auch während und nach der Gelierung auf. Der Gesamtschrumpf wird in einen physikalischen und einen chemischen Anteil unterteilt. Während der physikalische Schrumpf richtungsbestimmt ist und räumlich von den Außenbereichen des Polymerisats analog und im Einklang mit dem aufgebauten Temperaturgefälle nach innen zum Mittelpunkt des aushärtenden Formstoffs verläuft, ist der chemische Anteil nicht richtungsorientiert, resultiert einzig aus der Polymerbildung und ist stark von den geometrischen Konformations- und Konfigurationsparametern des neu aufgebauten Makromoleküls abhängig.

Bei lichthärtbaren Materialien können analoge Probleme auftreten. Man fand heraus, dass eine geringere Wärmefreisetzung und geringere Schrumpfkräfte dann auftreten, wenn es gelingt, die Polymerisation anfangs verzögert einsetzen zu lassen. Ein solcher Polymerisationsverlauf kann dadurch erreicht werden, wenn bei anfänglich geringerer Lichtleistung und späterer Anhebung der Lichtleistung auf einen Maximalwert, die Polymerisation bewusst verzögert wird. Durch die geringere Lichtleistung zu Beginn der Härtung bleibt das Material länger fließfähig, der Gelpunkt wird später erreicht, die Mobilität der funktionellen Gruppen bleibt länger erhalten, so dass höhere Umsatzraten erreicht werden. Die hier eingesetzte Technik wird "Soft-Start-Polymerisation" genannt. Sie ist neben der sogenannten "Inkrementaltechnik", bei der dünne Schichten einzeln lichtgehärtet werden (sehr zeitintensiv), eine weitere Technik, um hohe Wärmepeaks und den mit der Wärmefreisetzung gekoppelten Schrumpf reduzieren zu können.

Ansätze, schrumpfungsarme Dentalmaterialien zur Verfügung zu stellen, wie
- der Einsatz über ringöffnende Metathesepolymerisation (ROMP) härtender, bicyclischer Monomere (DE 199 05 093 A1),
- die Zugabe bestimmter Additive (Cumaronharz, Polyvinyacetat, Alkoholtenside) vor Aushärtung der Zusammensetzung (DE 198 51 038 A1)
- der Einsatz kationisch polymerisierbarer Oxetane (US 5,750,590)
- der Einsatz von Epoxidharzen, die nanoskalige anorganische Oxide enthalten sowie
- die Kombination unterschiedlich großer Füllstoffe (US 6,709,271 B1)
haben sich bisher in der Praxis nicht durchsetzen können.

Für autopolymerisierende und, in noch viel höherem Maße, für dualhärtende dentale Zusammensetzungen stellen Faktoren wie Wärmefreisetzung und Schrumpf Probleme dar, denen zurzeit in erster Linie durch Verfahren und weniger durch die chemische Rezeptur der dentalen Zusammensetzung begegnet wird.

In EP 1 720 506, WO 2009/083168 A1, DE 10 2008 028 306 A1, EP 1 872 767 A1 sowie EP 2 070 506 sind diverse polymerisierbare Dentalmaterialien beschrieben. Die dort offenbarten Dentalmaterialien können thermisch, chemisch, photochemisch und/oder durch Reaktion mit der Mund- oder Luftfeuchte ausgehärtet werden. Neben vielen anderen Stabilisatoren offenbaren diese Dokumente in der jeweiligen Liste der Stabilisatoren unter anderem Terpinene. Eine Kombination von photopolymerisierbaren Monomeren und Terpinenen ist in den genannten Dokumenten nicht offenbart.

Auf einem anderen dentalen Anwendungsgebiet, nämlich dem Gebiet der dentalen Abdeckmassen, wurde bei Systemen der radikalischen Polymerisation lichthärtbarer Massen das Problem der hohen Wärmeentwicklung in manchen Fällen dadurch angegangen, entweder der Zusammensetzung nichtreaktive organische Zusätze beizugeben und/oder Monomere hohen Molekulargewichts, d.h. Verbindungen mit einem geringen Doppelbindungsanteil, zu verwenden. Es seien insoweit die US 6,305,936 B1, US 6,800,671 B1 und WO 2008/096182 genannt.

DE 42 33 886 C1 offenbart polymerisierbare Konditionierungsmittel auf Methacryl-Basis und ein Verfahren zur Vorbehandlung der Oberfläche von Formkörpern aus Polyacrylat-, Polymethacrylat- und Polycarbonat-Kunststoffen vor dem Auftragen von polymerisierbarem Methacrylat-Material und die Verwendung des Konditionierungsmittels. Das Konditionierungsmittel kann monocyclischen Terpenkohlenwasserstoff enthalten. Ein intraoraler Einsatz erscheint aufgrund der Verwendung von als toxikologisch bedenklich einzustufenden Alkylmonomethacrylaten ausgeschlossen.

DE 27 27 480 A1 offenbart schlagzähe, glasartige Kunststofflegierungen aus Polymethacrylaten und aliphatischen Polyurethanharnstoffen, Es werden Verfahren zu deren Herstellung offenbart, in denen Monomerenmischungen eingesetzt werden, die schwefelhaltige Molekulargewichtsregler umfassen. Nicht offenbart werden Zusammensetzungen für den dentalen Bereich.

DE 17 95 395 A offenbart Verfahren zur Herstellung von Polymerisaten und Mischpolymerisaten von (Meth)Acrylsäureestern und von Acrylnitril in Gegenwart von Polymerisationskatalysatoren und von Verbindungen mit einem Sechserring, der zwei nichtkonjugierte Doppelbindungen aufweist, von denen eine semicyclisch (exocyclisch) sein kann. Die speziell offenbarten Polymerisationskatalysatoren sind thermische Katalysatoren. Photoinitiatoren oder photopolymerisierbare dentale Zusammensetzungen sind nicht offenbart.

US 4,490,497 A offenbart Pulver-Flüssigkeits-Systeme für einen chirurgischen Zement, die bei der extraoralen Herstellung dentaler Prothesen eingesetzt werden können. Eine Flüssigkomponente der Zusammensetzung umfasst als "chain stopper" beispielsweise ein doppelt ungesättigtes monocyclisches Terpen oder ein einfach ungesättigtes bicyclisches Terpen. Nicht offenbart sind dualhärtende Zusammensetzungen, insbesondere keine dualhärtenden dentalen Zusammensetzungen, die zum unmittelbaren Einsatz in der Mundhöhle geeignet sind. Ferner eignen sich die beschriebenen Pulver-Flüssigkeits-Systeme nicht zur Ausbringung mittels dentaler Spritzen. Die in US 4,490,497 A beschriebenen Systeme weisen zudem recht lange Abbindezeiten auf. Ein Zusammenhang zwischen dem Einsatz von "chain stopper" und Polymerisationsstabilisatoren (wie alkylsubstituierten Monophenolen) ist nicht offenbart.

DE 30 10 373 A1 offenbart Verfahren zur Polymerisation von Methacrylsäuremethylester oder dessen Gemischen mit weiteren Vinylmonomeren in Gegenwart von Enolethern. Die nach dem offenbarten Verfahren hergestellten Polymerisate werden zwar als besonders geeignet für den Einsatz im Dentalbereich bezeichnet, beispielsweise zur Herstellung von Prothesen nach dem Pulver-Flüssigkeitsverfahren, nicht offenbart sind jedoch dualhärtende mehrkomponentige Zusammensetzungen.

EP 1 720 506 B1 offenbart ein gefülltes und polymerisierbares Dentalmaterial sowie ein Verfahren zu dessen Herstellung. Als Anwendungsbeispiele werden genannt: Zahnfüllungsmaterialien, Stumpfaufbaumaterialien, Materialien für provisorische Kronen und Brücken, Zahnzemente, Adhäsive, Materialien für künstliche Zähne, Verblendmaterialien, Versiegelungsmaterialien und Dentallacke. Es wird offenbart, dass das Dentalmaterial zur Einstellung bestimmter Eigenschaften Additive bzw. Modifikatoren enthalten kann; in einer langen Liste von Beispielen findet sich auch "Terpinene". Nicht offenbart werden allerdings dualhärtende, mehrkomponentige Zusammensetzungen, welche einen Molekulargewichtsregler umfassen. Das Dokument EP 2 072 028 beschreibt dualhärtende, mehrkomponentige dentale Zusammensetzungen umfassend: mehrere photopolymerisierbare (Meth)Acrylaten, ein oder mehrere Photoinitiatoren, ein Polymerisationsinhibitor (BHT), mehrere anorganische Füllstoffe, ein oder mehrere Initiatoren für eine chemische Aushärtung bei Umgebungstemperatur.

Der Fachmann, z.B. der Zahnarzt, wünscht eine (möglichst) lange extraorale Verarbeitungszeit und anschließend eine möglichst kurze intraorale Abbindezeit nach dem Einbringen in den Mund (also in der Regel bei leicht erhöhter Temperatur). Dabei darf die Aushärtung nicht mit einer großen Exothermie einhergehen, da bei zu hohen Temperaturen lebende Zahnsubstanz nachhaltig geschädigt werden kann.

Um Verarbeitungszeit und Abbindezeit zu beeinflussen, kann eine dentale Zusammensetzung variiert werden. Erhöht man bei ansonsten gleicher Zusammensetzung beispielsweise die zugegebene Menge eines Polymerisationsinhibitors in einer radikalisch polymerisierbaren Zusammensetzung, so verlängert sich regelmäßig sowohl die Verarbeitungszeit als auch die Abbindezeit, wobei sich die Verzögerung der Härtung in etwa proportional zu der zugegebenen Menge an Polymerisationsinhibitor verhält.

Es war die Aufgabe der Erfindung, dentale dualhärtende (dentale chemisch- und gleichzeitig lichthärtende) Zusammensetzungen zur Verfügung zu stellen, die gegenüber den Zusammensetzungen aus dem Stand der Technik eine geringere Wärmeentwicklung und einen geringeren Schrumpf aufweisen.

Eine spezielle dentale Aufgabenstellung bestand darin, eine gefüllte dualhärtende dentale Zusammensetzung bereitzustellen (insbesondere ein Kompositmaterial, welche als dentales Füllungsmaterial, als fließfähiges Kompositmaterial, als Kronenmaterial, als Brückenmaterial und/oder als Stumpfaufbaumaterial verwendbar ist), welche mit einer dentalen Spritze applizierbar sein sollte und welche vorzugsweise in Form zweier Pasten, d.h. in Form eines Paste-Paste-Systems, vorliegen sollte.

Ferner sollte die Verarbeitungszeit ausreichend lang und gleichzeitig die Abbindezeit möglichst kurz sein. Dabei sollte trotz möglichst kurzer Abbindezeit eine möglichst geringe Temperaturentwicklung stattfinden, d.h. ein akzeptables Temperaturmaximum bei der Aushärtung der dentalen Zusammensetzung im Mundraum nicht überschritten werden.

Die vorliegende Erfindung betrifft eine dualhärtende, mehrkomponentige dentale Zusammensetzung umfassend:
(a) ein oder mehrere photopolymerisierbare Monomere ausgewählt aus der Gruppe bestehend aus Acrylaten und Methacrylaten, vorzugsweise der Gruppe der Methacrylate,
(b) ein oder mehrere Photoinitiatoren, ausgewählt aus der Gruppe bestehend aus alpha-Diketonen, Benzoinalkylethern, Thioxanthonen, Benzophenonen, Acylphosphinoxiden, Acetophenonen, Ketalen, Titanocenen, Boraten und sensibilisierenden Farbstoffen,
(c) ein oder mehrere Molekulargewichtsregler ausgewählt aus der Gruppe bestehend aus Verbindungen, die mit einem Radikal eines Monomers des Bestandteils (a) umsetzbar sind, wobei die Umsetzung unter Abstraktion eines H-Radikals aus dem Molekulargewichtsregler in Allylposition erfolgt,
   und
   wobei ein oder mehrere der Molekulargewichtsregler des Bestandteils (c) Verbindungen mit zwei oder mehr Doppelbindungen sind, die durch Abstraktion eines Allyl-H-Atoms ein delokalisiertes Elektronensystem ausbilden können, das sich über 5 oder mehr C-Atome erstreckt,
   und ausgewählt sind aus der Gruppe der Monoterpene α-Terpinen, β-Terpinen, γ-Terpinen, α-Phellandren, *β*-Phellandren und Terpinolen, oder ausgewählt sind aus der Gruppe bestehend aus Linolsäure, Linolensäure und sonstigen substituierten oder nicht substituierten Cyclohexadienen, bevorzugt ausgewählt aus der Gruppe bestehend aus α-Terpinen und γ-Terpinen,
(d) ein oder mehrere Polymerisationsinhibitoren zur Erhöhung der Lagerstabilität der Zusammensetzung, ausgewählt aus der Gruppe bestehend aus Hydrochinonmonomethylether (HQME), 2,6-Di-tert.butyl-4-methylphenol (BHT), tert.-Butylhydroxyanisol (BHA), 2,2-Diphenyl-1-picrylhydrazyl-Radikalen, Galvinoxyl-Radikalen, Triphenylmethyl-Radikalen, 2,3;6,6,-Tetramethylpiperidinyl-1-oxylradikal (TEMPO) und Phenothiazin,
(e) ein oder mehrere anorganische Füllstoffe,
(f) ein oder mehrere Initiatoren für eine chemische Aushärtung bei Umgebungstemperatur (vorzugsweise 23 °C), vorzugsweise ein Redoxinitiatorsystem,
sowie gegebenenfalls ein oder mehrere weitere Additive.

Überraschenderweise wurde gefunden, dass es gelingt, dentale Zusammensetzungen auf der Basis photopolymerisierbarer Monomere aus der Gruppe der Acrylate und Methacrylate in Form von erfindungsgemäßen dualhärtenden, mehrkomponentigen dentalen Zusammensetzungen zur Verfügung zu stellen, die keine großen Wärmemengen an die Umgebung abgeben und einen geringen physikalischen Schrumpf sowie hohe Vernetzungsraten aufweisen und nach Aushärtung des Materials keine nachteiligen Mengen nicht umgesetzter Monomere mehr enthalten, die später einen schädlichen Einfluss auf das Gewebe nehmen können.

Überraschenderweise wurde zudem gefunden, dass eine Kombination von einem oder mehreren Molekulargewichtsreglern des Bestandteils (c) und einem oder mehreren Polymerisationsinhibitoren des Bestandteils (d) bei den dualhärtenden erfindungsgemäßen dentalen Zusammensetzungen zu einem solchen Aushärtungsverhalten und einer solchen Polymerisationskinetik führt, dass nach dem Vermischen (z.B. in einem statischen Mischrohr) eine lange extraorale Verarbeitung des Dentalwerkstoffs möglich ist (vgl. die Anmerkungen zur Verarbeitungszeit weiter unten), jedoch nach intraoraler Applikation eine schnelle Aushärtung und Abbindung stattfindet (vgl. die Anmerkungen zur Abbindezeit weiter unten).

Da die erfindungsgemäßen Zusammensetzungen dualhärtend sind, wird die Polymerisation bzw. die Aushärtung üblicherweise durch Belichtung weiter beschleunigt.

Kurz zusammengefasst kann durch die erfindungsgemäßen Zusammensetzungen, insbesondere in einer der nachfolgend als bevorzugt bzw. besonders bevorzugt gekennzeichneten Ausgestaltungen, somit innerhalb eines gewissen Rahmens eine vergleichsweise lange Verarbeitungszeit erreicht werden, ohne dass dabei auch die Abbindezeit entsprechend verlängert würde.

Die Begriffe und Definition von "Verarbeitungszeit" und "Abbindezeit" sowie die Methoden zu deren Bestimmung beziehen sich im Rahmen des vorliegenden Textes auf die DIN EN ISO 4049 (Stand: März 2010).

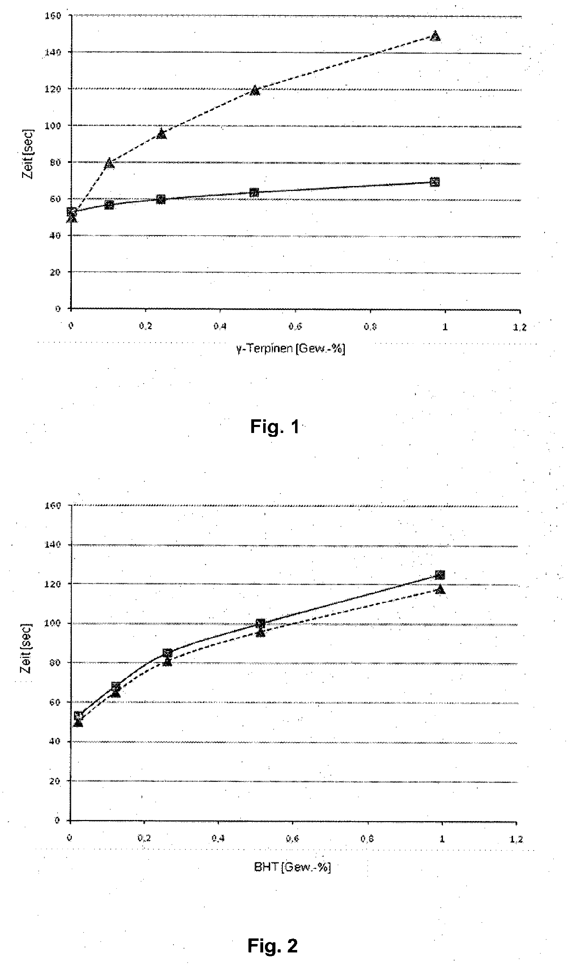

Die Verarbeitungszeiten und Abbindezeiten für verschiedene erfindungsgemäße dualhärtende, zweikomponentige dentale Zusammensetzungen enthaltend 0,02 Gew.-% des Polymerisationsinhibitors BHT (2,6-Di-tert.butyl-4-methylphenol; Verbindung des Bestandteils (d)) und unterschiedliche Mengen an γ-Terpinen (Verbindung des Bestandteils (c)) (bei ansonsten identischer Zusammensetzung) sind in Fig.1 illustriert.

Die Verarbeitungszeiten und Abbindezeiten für verschiedene nicht erfindungsgemäße dualhärtende, zweikomponentige dentale Zusammensetzungen enthaltend unterschiedliche Mengen des Polymerisationsinhibitors BHT (bei ansonsten identischer Zusammensetzung) in Abwesenheit eines Molekulargewichtsreglers gemäß Bestandteil (c) sind in Fig.2 dargestellt.

Gemäß der Methode der DIN EN ISO 4049 wird die Verarbeitungszeit bei einer (Form-)Temperatur von 23°C bestimmt und die Abbindezeit bei 37°C. Diese (Form-)Temperaturen entsprechen üblichen extraoralen bzw. intraoralen Bedingungen.

Fig.1 illustriert die Verarbeitungszeiten und Abbindezeiten bei einer konstanten Menge an BHT in Abhängigkeit des Gehaltes von γ-Terpinen einer ansonsten identischen erfindungsgemäßen dentalen Zusammensetzung. Die Abszisse von Fig.1 gibt den prozentualen Gehalt an γ-Terpinen in der jeweiligen Zusammensetzung an, die Ordinate die Zeit in Sekunden. Die ermittelten Verarbeitungszeiten entsprechen der gestrichelten Linie mit Dreiecken, die ermittelten Abbindezeiten der durchgezogenen Linie mit Quadraten.

Fig.2 illustriert beispielhaft die Verarbeitungszeiten und Abbindezeiten in Abhängigkeit vom Gehalt an BHT in einer ansonsten identischen Zusammensetzung. Die Abszisse von Fig.2 gibt den prozentualen Gehalt an BHT in der jeweiligen Zusammensetzung an, die Ordinate die Zeit in Sekunden. Die ermittelten Verarbeitungszeiten entsprechen der gestrichelten unteren Linie mit Dreiecken, die ermittelten Abbindezeiten der durchgezogenen oberen Linie mit Quadraten.

Aus Fig.1 ist unmittelbar erkennbar, dass die Abbindezeit von der Verarbeitungszeit mit steigendem Gehalt an γ-Terpinen zunehmend abweicht, also sozusagen eine Entkopplung von Abbindezeit und Verarbeitungszeit erfolgt.

Aus Fig.2 hingegen ist unmittelbar erkennbar, dass der Kurvenverlauf für die Abbindezeitwerte dem für die Verarbeitungszeitwerte qualitativ entspricht und - in den konkret untersuchten Beispielen - die Abbindezeit jeweils geringfügig länger ist als die entsprechende Verarbeitungszeit.

Die in den Figuren 1 und 2 dargestellten Ergebnisse resultieren aus eigenen Untersuchungen mit Zusammensetzungen entsprechend bzw. basierend auf der Rezeptur des Referenzbeispiels und den Rezepturen der erfindungsgemäßen Beispiele Exp. 1 bis Exp. 4. Genauere Angaben hierzu finden sich weiter unten (siehe auch die nachfolgenden Tabellen 1, 1A-1, 1A-2 und 1B).

Eine bevorzugte erfindungsgemäße dentale Zusammensetzung weist eine Abbindezeit (gemäß DIN EN ISO 4049, wie oben spezifiziert) von höchstens 10 Minuten auf, bevorzugt von höchstens 6 Minuten, weiter bevorzugt von höchstens 3 Minuten.

Eine bevorzugte erfindungsgemäße dentale Zusammensetzung ist zweikomponentig, und liegt vorzugsweise in Form zweier Pasten vor.

Erfindungsgemäße dualhärtende Zusammensetzungen liegen im Allgemeinen in Form einer Basispaste und einer Katalysatorpaste vor. Eine bevorzugte erfindungsgemäße dentale Zusammensetzung ist zweikomponentig und liegt in Form einer Basispaste und einer Katalysatorpaste vor, wobei vorzugsweise die gewichtsbezogenen Mischungsverhältnisse von Basispaste zu Katalysatorpaste im Bereich von 1 : 2 bis 10 : 1 liegen, bevorzugt im Bereich von 1 : 1 bis 10 : 1.

In einer bevorzugten Ausgestaltung ist Bestandteil (d) teilweise oder vollständig in der Katalysatorpaste einer erfindungsgemäßen dentalen Zusammensetzung enthalten.

Eine bevorzugte erfindungsgemäße dentale Zusammensetzung ist mit einer dentalen Spritze, insbesondere mit einer Doppelspritze oder Doppelkammerkartusche, über eine Kanüle, vorzugsweise ein statisches Mischrohr, ausbringbar und applizierbar.

Bevorzugt sind dabei Doppelkammerspritzen und Doppelkammerkartuschen mit Mischaufsatz, da mit diesen ein schnelles und blasenfreies Anmischen in genau definierten Mischungsverhältnissen möglich ist.

Eine bevorzugte erfindungsgemäße dentale Zusammensetzung weist bei Applikation im Mund ein Fließverhalten und eine solche Standfestigkeit auf, dass ein Herunterlaufen oder Spreiten der applizierten dentalen Zusammensetzung nicht auftritt.

Vorzugsweise ist die Viskosität zumindest einer Komponente, bevorzugt jedoch die Viskosität jeder Komponente der erfindungsgemäßen dentalen Zusammensetzung größer oder gleich 10 Pas und liegt bevorzugt im Bereich von 10 Pas bis 190 Pas, besonders bevorzugt im Bereich von 20 bis 140 Pas.

Bei höheren Viskositätswerten, insbesondere oberhalb von 190 Pas, wird das Anfließverhalten der Zusammensetzung signifikant schlechter und ist aus einer üblichen dentalen Mischkanüle nur noch sehr schlecht ausdrückbar.

Dentale Zusammensetzungen mit einer Viskosität im ganz besonders bevorzugten Bereich von 20 bis 140 Pas lassen sich unter Verwendung üblicher dentaler Mischkanülen besonders komfortabel und in sowohl für den Patienten als auch für den behandelnden Zahnarzt angenehmer Weise in der Mundhöhle applizieren, ohne dass es zu unerwünschter Tropfenbildung oder zu einem Wegfließen kommt.

Die hier angegebenen Viskositäten beziehen sich auf die Messung bei 23°C mit einem Platte-Platte-Rheometer (Durchmesser der Platte: 25 mm) mit einer Scherrate von 10/sec. bei einer Spaltbreite von 1 mm.

Erfindungsgemäß ebenfalls bevorzugt ist eine dentale Zusammensetzung, wobei Bestandteil (f) umfasst:
(f-a)
   - ein oder mehrere Initiatoren, die ausgewählt sind aus der Gruppe bestehend aus anorganischen Peroxiden, organischen Peroxiden, anorganischen Hydroperoxiden, organischen Hydroperoxiden, Barbitursäurederivaten, Malonylsulfamiden, Protonensäuren, Lewis- oder Broensted-Säuren, solche Säuren freisetzenden Verbindungen und Carbeniumionen-Donatoren, wie vorzugsweise Methyltriflat oder Triethylperchlorat;
   - ein oder mehrere Co-Initiatoren ausgewählt aus der Gruppe bestehend aus
   tertiären Aminen, Schwermetallverbindungen, insbesondere Verbindungen der 8. und der 9. Gruppe des Periodensystems ("Eisen- und Kupfergruppe"), Verbindungen mit ionogen gebundenen Halogenen oder Pseudohalogenen, vorzugsweise quartären Ammoniumhalogeniden, und schwachen Broenstedt-Säuren, wie z. B. Alkohole und Wasser,
   und/oder
(f-b) ein Redoxinitiatorsystem, umfassend
(f-b1) Barbitursäure, Thiobarbitursäure, ein Barbitursäure- und/oder ein Thiobarbitursäurederivat,
(f-b2) gegebenenfalls eine Peroxodisulfat- und/oder eine Peroxodiphosphatverbindung,
(f-b3) eine Kupferverbindung,
(f-b4) eine Verbindung mit einem ionogen vorliegenden Halogenatom,
wobei die Initiatoren des Bestandteils (f) so auf separate Komponenten der dentalen Zusammensetzung verteilt sind, dass eine chemische Initiierung durch Vermischen der besagten Komponenten ausgelöst wird.

Dem Fachmann sind diverse Initiatorsysteme für eine chemische Aushärtung sowie auch entsprechende Redoxinitiatorsysteme bekannt. Es sei insoweit exemplarisch auf die EP 1 720 506 und insbesondere die EP 1 839 640 sowie die darin zitierten Dokumente verwiesen.

Molekulargewichtsregler (im Folgenden auch kurz als Regler bezeichnet) sind an sich bekannt und kommerziell erhältlich. Man benutzt sie beispielsweise bei der Lösungspolymerisation von Olefinen, bei der Emulsionspolymerisation von Methacrylaten oder zur Herstellung von Formkörpern aus PMMA-Formmassen (PMMA = Polymethylmethacrylat) durch Formpressen oder Spritzgießen. Molekulargewichtsregler stellen sogenannte Übertragungsreagentien dar, die in einer freien radikalischen Reaktion Transferreaktionen eingehen und mechanistisch H-Abstraktion und Übertragung der Radikalfunktion auf den Regler beinhaltet. Der Regler findet sich dann aufgrund seiner Funktion in Form von Endgruppen im vernetzten Polymerisat wieder.

Übliche Regler sind beispielsweise Aldehyde und Ketone, wie Formaldehyd, Acetaldehyd, Propionaldehyd, *n*-Butyraldehyd, Isobutyraldehyd, Methylethylketon, Aceton, Methylisobutylketon, Ameisensäure, Ammoniumformiat, Hydroxylammoniumsulfat und Hydroxylammoniumphosphat, Verbindungen, die Schwefel in organisch gebundener Form enthalten, wie Di-*n*-butylsulfid, Di-*n-*octylsulfid, Diphenylsulfid, Düsopropyldisulfid, Di-*n*-butyldisulfid, Di-*n*-hexyldisulfid, Diacetyldisulfid und Di-tert.-butyltrisulfid, Verbindungen, die Schwefel in Form von SH-Gruppen aufweisen, wie *n*-Butylmercaptan, *n*-Hexylmercaptan und *n*-Dodecylmercaptan, Octadecylmercaptan, weitere Schwefelverbindungen wie Hydrogensulfite, Disulfite, Verbindungen wie Mercaptoethanol, Mercaptobutanol, Mercaptoessigsäure, 3-Mercaptopropionsäure, Mercaptobernsteinsäure, Thioglycerin, Thioglykolsäure, Diethanolsulfid, Thiodiglykol, Ethylthioethanol, 2,2,4,6,6-Pentamethylheptan-4-thiol, 2,2,4,6,6,8,8-Heptamethylnonan-4-thiol, Thioharnstoff, Dimethylsulfoxid, Ethylhexylthioglycolat, Pentaerythrittetrathioglycolat, Mercaptopropyltrimethoxysilan, dann Allylverbindungen wie Allylalkohol, Allylbromid, oder Benzylverbindungen wie Benzylchlorid oder Alkylhalogenide wie Chloroform, Bromtrichlormethan oder Tetrachlormethan, Tetrabrommethan, Methylenchlorid, weiterhin niedere und höhermolekulare einwertige oder mehrwertige Alkohole wie Methanol, Ethanol, *n*-Propanol, Isopropanol, tert.-Butanol, sec-Butanol, *n*-Butanol, Amylalkohol, Cyclohexanol, Octanol, Dodecanol, 1-Ethylhexanol, Glycerin, Stearylalkohol, Oleylalkohol, Hydroxyethylmethacrylat oder Amine wie Triethylamin sowie Toluol oder Ethylbenzol.

Die oben genannten Regler sind für den hier vorgesehenen Einsatz jedoch ungeeignet. So weisen schwefelhaltige Molekulargewichtsregler einen intensiven und typisch unangenehmen Geruch auf, sie scheiden als Bestandteil eines dentalen Materials aus. Erfindungsgemäße dentale Zusammensetzungen umfassen daher vorzugsweise keinen schwefelhaltigen Molekulargewichtsregler. Ferner wurde gefunden, dass Alkohole, nieder- oder höhermolekular, ein-oder mehrwertig, in den Zusammensetzungen keine regelnde Wirkung entfalten und unwirksam bleiben. Gleiches gilt für Aldehyde und Ketone.

Der genaue Grund für den erfindungsgemäßen Erfolg bei Verwendung der oben angegebenen Molekulargewichtsregler (c) ist nicht im Detail bekannt. Vermutlich besteht jedoch eine besondere, vorab nicht zu erwartende, Wechselwirkung zwischen dem oder den Monomeren und dem oder den Molekulargewichtsreglern, die es ermöglicht, dass bei der Photopolymerisation der erfindungsgemäßen Zusammensetzung der Molekulargewichtsregler (c) in einer Art und Weise und in einem Maße Einfluss auf den Polymerisationsverlauf nimmt, dass Kettenlänge, Vernetzung und Restmonomergehalt des sich bildenden Polymers derart gestaltet sind, dass die gewünschten Eigenschaften erhalten werden.

Erfindungsgemäß geeignete Molekulargewichtsregler sind beispielsweise α-Terpinen, β-Terpinen, γ-Terpinen, α-Phellandren, β-Phellandren und Terpinolen (auch δ-Terpinen, genannt), 1,4-Cyclohexadien (gegebenenfalls substituiert), 1,3-Cyclohexadien (gegebenenfalls substituiert), sowie Linolsäure und α-Linolensäure.

Erfindungsgemäß als Bestandteil (c) einer erfindungsgemäßen Zusammensetzung einzusetzende Molekulargewichtsregler sind Verbindungen mit zwei oder mehr Doppelbindungen, die durch Abstraktion eines Allyl-H-Atoms ein delokalisiertes Elektronensystem ausbilden können, das sich über 5 oder mehr C-Atome erstreckt.

Erfindungsgemäß als Bestandteil (c) einer erfindungsgemäßen Zusammensetzung einzusetzende Molekulargewichtsregler sind dabei ausgewählt aus der Gruppe der Monoterpene α-Terpinen, β-Terpinen, γ-Terpinen, α-Phellandren, β-Phellandren und Terpinolen oder sind ausgewählt aus der Gruppe bestehend aus Linolsäure, Linolensäure und sonstigen substituierten oder nicht substituierten Cyclohexadienen.

Erfindungsgemäß besonders bevorzugte Regler sind α-Terpinen und γ-Terpinen, insbesondere bevorzugt ist γ-Terpinen. Mit diesen Molekulargewichtsreglern wurden die besten Ergebnisse im Sinne der vorliegenden Erfindung erzielt.

In einer erfindungsgemäßen Zusammensetzung können selbstverständlich auch Mischungen von Molekulargewichtsreglern eingesetzt werden.

Erfindungsgemäß bevorzugte dentale Zusammensetzungen umfassen

Bestandteil (a) in einer Menge von 19 bis 70 Gew.-%, vorzugsweise in einer Menge von 30 bis 65 Gew.-%,

Bestandteil (c) in einer Menge von 0,05 bis 5,00 Gew.-%, vorzugsweise von 0,1 bis 2,0 Gew.-%, bevorzugt von 0,1 bis 0,5 Gew.-%, und

Bestandteil (e) in einer Menge von 25 bis 80 Gew.-%, vorzugsweise von 30 bis 65 Gew.-%,

jeweils bezogen auf die Gesamtmasse der dentalen Zusammensetzung.

Erfindungsgemäß bevorzugte dentale Zusammensetzungen umfassen

Bestandteile (b) und (f) in einer Gesamtmenge im Bereich von 0,25 bis 3 Gew.-%, vorzugsweise in einer Gesamtmenge im Bereich von 0,35 bis 1,5 Gew.-%, bezogen auf die Gesamtmasse der dentalen Zusammensetzung.

Erfindungsgemäß bevorzugte dentale Zusammensetzungen umfassen

Bestandteil (e) in einer Menge von zumindest 10 Gew.-%, vorzugsweise von zumindest 20 Gew.-%, bevorzugt von zumindest 25 Gew.-%, und vorzugsweise gleichzeitig

Bestandteile (b) und (f) in einer Gesamtmenge im Bereich von 0,25 bis 3 Gew.-%, vorzugsweise in einer Gesamtmenge im Bereich von 0,35 bis 1,5 Gew.-%, und/oder vorzugsweise gleichzeitig

Bestandteil (d) in einer Menge 0,005 bis 0,100 Gew.-%, vorzugsweise von 0,005 bis 0,075 Gew.-%, bevorzugt von 0,010 bis 0,050 Gew.-%,

jeweils bezogen auf die Gesamtmasse der dentalen Zusammensetzung.

Erfindungsgemäß weiter bevorzugte dentale Zusammensetzungen umfassen

Bestandteil (a) in einer Menge von 19 bis 70 Gew.-%, vorzugsweise in einer Menge von 30 bis 65 Gew.-%,

Bestandteil (c) in einer Menge von 0,05 bis 5,00 Gew.-%, vorzugsweise von 0,1 bis 2,0 Gew.-%, bevorzugt von 0,1 bis 0,5 Gew.-%,

Bestandteil (e) in einer Menge von 25 bis 80 Gew.-%, vorzugsweise von 30 bis 65 Gew.-%, und

Bestandteile (b) und (f) in einer Gesamtmenge im Bereich von 0,25 bis 3 Gew.-%, vorzugsweise in einer Gesamtmenge im Bereich von 0,35 bis 1,5 Gew.-%,

jeweils bezogen auf die Gesamtmasse der dentalen Zusammensetzung.

Erfindungsgemäß weiter bevorzugte dentale Zusammensetzungen umfassen

Bestandteil (a) in einer Menge von 19 bis 70 Gew.-%, vorzugsweise in einer Menge von 30 bis 65 Gew.-%,

Bestandteil (b) in einer Menge von 0,05 bis 1,00 Gew.-%, vorzugsweise von 0,05 bis 0,5 Gew.-%, bevorzugt von 0,05 bis 0,2 Gew.-%,

Bestandteil (c) in einer Menge von 0,05 bis 5,00 Gew.-%, vorzugsweise von 0,1 bis 2,0 Gew.-%, bevorzugt von 0,1 bis 0,5 Gew.-%,

Bestandteil (e) in einer Menge von 25 bis 80 Gew.-%, vorzugsweise von 30 bis 65 Gew.-%, und Bestandteil (f) in einer Menge von 0,2 bis 2 Gew.-%, vorzugsweise von 0,3 bis 1,0 Gew.-%,

jeweils bezogen auf die Gesamtmasse der dentalen Zusammensetzung.

Im Rahmen des vorliegenden Textes bezeichnet die "Gesamtmasse der dentalen Zusammensetzung" in einem mehrkomponentigen System die Gesamtmasse sämtlicher Komponenten der dentalen Zusammensetzung.

Eine bevorzugte erfindungsgemäße dentale Zusammensetzung enthält neben einem oder mehreren Photoinitiatoren des Bestandteils (b) zusätzlich einen oder mehrere von Bestandteil (b) verschiedene Initiatoren für eine chemische Aushärtung bei Umgebungstemperatur (vorzugsweise 23 °C) des Bestandteils (f).

### Bestandteil (a) - photopolymerisierbare Monomere

Die radikalisch lichtpolymerisierbaren Monomere können mindestens zwei ethylenische Gruppen aufweisende Substanzen sein wie beispielsweise, ohne darauf beschränkt zu sein, die in der Dentalchemie üblicherweise in Kompositmaterialien eingesetzten (Meth)acrylatmonomere.

In einer Vielzahl erfindungsgemäßer Zusammensetzungen wird vorzugsweise auf die Anwesenheit von Methylmethacrylat verzichtet werden, da Methylmethacrylat als toxikologisch bedenklich eingeschätzt wird. Eine erfindungsgemäße dentale Zusammensetzung enthält daher vorzugsweise höchstens 5 Gew.-% Methylmethacrylat, bevorzugt höchstens 2 Gew.-%, weiter bevorzugt höchstens 1 Gew.-%, besonders bevorzugt höchstens 0,5 Gew.-%, jeweils bezogen auf das Gesamtgewicht der Zusammensetzung. In einer weiter bevorzugten Ausgestaltung ist eine erfindungsgemäße dentale Zusammensetzung frei von Methylmethacrylat.

In einer bevorzugten Ausgestaltung enthält Bestandteil (a) einer erfindungsgemäßen dentalen Zusammensetzung keine Alkylmonomethacrylate. In einer weiteren bevorzugten Ausgestaltung enthält eine erfindungsgemäße dentale Zusammensetzung höchstens 5 Gew.-% an Alkylmonomethacrylaten, vorzugsweise höchstens 2 Gew.-%, bevorzugt höchstens 1 Gew.-%, weiter bevorzugt höchstens 0,5 Gew.-%. Besonders bevorzugt ist eine erfindungsgemäße dentale Zusammensetzung frei von Alkylmonomethacrylaten.

In der Patentliteratur ist eine Vielzahl weiterer Verbindungen genannt (beispielsweise auch in der DE 39 41 629 A1, die allesamt Diester der Acryl- oder Methacrylsäure sind und sich zum Einsatz in einer erfindungsgemäßen Zusammensetzung eignen.

In einer bevorzugten erfindungsgemäßen dentalen Zusammensetzung enthält Bestandteil (a) ein oder mehrere Dimethacrylat-Monomere gewählt aus der Gruppe bestehend aus Ethylenglykoldimethacrylat (EGDMA), 1,6-Hexandioldimethacrylat (HEDMA), Triethylenglykoldimethacrylat (TEGDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat, Bisphenol-A-Glycidyl-Methacrylat (Bis-GMA) und Dimethacrylaten des Dihydroxymethyltricyclo[5.2.1.0^{2,6}]decans.

Explizit bevorzugt sind auch die entsprechenden Dimethacrylate bzw. Diacrylate des Dihydroxymethyltricyclo[5.2.1.0^{2,6}]decans, wie in den Druckschriften DE 1816823, DE 2419887, DE 2406557, DE 2931926, DE 3522005, DE 3522006, DE 3703120, DE 102005021332, DE 102005053775, DE 102006060983, DE 69935794 und DE 102007034457 beschrieben.

Die radikalisch lichtpolymerisierbaren Monomere können auch mindestens eine ethylenische Doppelbindung aufweisende Hydroxylverbindungen sein. Dabei können alle in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

In einer weiteren bevorzugten Ausgestaltung besteht Bestandteil (a) einer erfindungsgemäßen dentalen Zusammensetzung aus Monomeren ausgewählt aus der Gruppe bestehend aus (Meth)acrylatmonomeren mit mindestens zwei ethylenischen Gruppen und Hydroxylverbindungen von Acrylaten oder Methacrylaten, oder Bestandteil (a) umfasst solche Monomere.

In einer weiteren bevorzugten Ausgestaltung besteht Bestandteil (a) einer erfindungsgemäßen dentalen Zusammensetzung aus Monomeren ausgewählt aus der Gruppe bestehend aus Methacrylatmonomeren mit mindestens zwei ethylenischen Gruppen und Hydroxylverbindungen von Methacrylaten, oder Bestandteil (a) umfasst solche Monomere.

Ferner sind die sogenannten Ormocere, die den Fachleuten bekannt sind und die beispielsweise in der DE 199 03 177 oder in der DE 44 16 857 beschrieben sind, verwendbar.

Eine erfindungsgemäße Zusammensetzung kann ferner eine oder mehrere säuregruppenhaltige lichthärtbare Substanzen enthalten. Säuregruppenhaltige Monomere können vorzugsweise eine Carbonsäure-, eine Phosphorsäure-, eine Phosphonsäure-, eine Sulfonsäure- und/oder eine Thiophosphorsäurefunktion aufweisen.

Die säuregruppenhaltige, lichthärtbare Verbindung kann ein polymerisierbares Monomer sein, das eine oder mehrere Säurefunktionen in einem Molekül enthält.

Geeignete eine Phosphorsäuregruppe enthaltende Monomere sind beispielsweise 2-(Meth)acryloyloxyethyldihydrogenphosphat, Bis[2-(meth)acryloyloxyethyl]hydrogenphosphat, 2-(Meth)acryloyloxyethylphenylhydrogenphosphat, 6-(Meth)-acryloyloxyhexyldihydrogenphosphat, 10-(Meth)acryloyloxydecyldihydrogenphosphat (MDP), 6-(Meth)-acryloyloxyhexylphenylhydrogenphosphat, 10-(Meth)acryloyloxydecyldihydrogenphosphat, 1,3-Di-(meth)acryloyloxypropan-2-dihydrogenphosphat, 1,3-Di-(meth)acryloyloxypropan-2-phenylhydrogenphosphat und Bis[5-(2-(meth)acryloyloxyethoxycarbonyl)heptyl]hydrogenphosphat.

Geeignete eine Carbonsäuregruppe enthaltende Monomere sind beispielsweise 4-(Meth)acryloxyethyltrimellithsäure (4-MET), 4-(Meth)acryloxyethyltrimellithsäureanhydrid (4-META), 4-(Meth)acryloxydecyltrimellithsäure, 4-(Meth)acryloxydecyltrimellithsäureanhydrid, 11-(Meth)acryloyloxy-1,1-undecandicarbonsäure, 1,4-Di(meth)acryloyloxypyromellithsäure, 2-(Meth)acryloyloxyethylmaleinsäure, 2-(Meth)acryloyloxyethylphthalsäure und 2-(Meth)acryloyloxyethylhexahydrophthalsäure.

Weitere geeignete säuregruppentragende Monomere sind beispielsweise in der EP 0 980 682 B1 oder der EP 0 948 955 genannt.

Ferner können auch die Phosphorsäureester mit Glycerindimethacrylat oder mit Hydroxyethylmethacrylat oder mit Hydroxypropylmethacrylat verwendet werden.

Die genannten lichtpolymerisierbaren Monomere können einzeln oder in Gemischen eingesetzt werden.

### Bestandteil (b) - Photoinitiatoren

Beispiele für einen Lichthärtungsinitiator schließen Katalysatoren ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer erfindungsgemäßen Zusammensetzung bewirken können.

Das Absorptionsmaximum von Campherchinon (CC) liegt bei ca. 470 nm und somit im Bereich des blauen Lichts. Campherchinon (CC) zählt zu den PI₂-Initiatoren und wird regelmäßig zusammen mit einem Coinitiator eingesetzt.

Vorzugsweise enthält eine erfindungsgemäße Zusammensetzung die Kombination eines alpha-Diketons und eines aromatischen tertiären Amins, bevorzugt ist die Kombination von Campherchinon (CC) und Ethyl-*p*-*N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einer erfindungsgemäßen Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem in den erfindungsgemäßen Zusammensetzungen.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. 11, Elsevier Applied Science, London, New York 1993 beschrieben.

### Bestandteil (f) - Initiatoren für die chemische Aushärtung

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid (BPO), Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonysulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Geeignete Malonysulfamide sind in der EP 0 059 451 beschrieben. Bevorzugte Verbindungen sind dabei 2,6-Dimethyl-4-isobutylmalonylsulfamid, isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2, 6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

### Bestandteil (d) - Polymerisationsinhibitoren

Die erfindungsgemäßen dentalen Zusammensetzungen enthalten einen oder mehrere Inhibitoren, auch Stabilisatoren genannt. Diese werden einer Zusammensetzung zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Hydrochinonmonomethylether (HQME) oder 2,6-Di-tert.butyl-4-methylphenol (BHT). Weitere Inhibitoren wie 2,2-Diphenyl-1-picrylhydrazyl-, Galvinoxyl-, Triphenylmethyl-Radikale, 2,3,6,6,-Tetramethylpiperidinyl-1-oxylradikale (TEMPO) sowie Phenothiazin werden in der EP 0 783 880 B1 beschrieben. Alternative Inhibitoren sind in der DE 101 19 831 A1 oder in der EP 1 563 821 A1 angegeben.

Eine erfindungsgemäße dentale Zusammensetzung umfasst ferner
(d) ein oder mehrere Polymerisationsinhibitoren zur Erhöhung der Lagerstabilität der Zusammensetzung, ausgewählt aus der Gruppe bestehend aus Hydrochinonmonomethylether (HQME), 2,6-Di-tert.butyl-4-methylphenol (BHT), tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) und Phenothiazin.

Erfindungsgemäß sind als Bestandteil (d) einer erfindungsgemäßen Zusammensetzung einzusetzende Molekulargewichtsregler ausgewählt aus der Gruppe bestehend aus 2,6-Di-tert.butyl-4-methylphenol (BHT) und tert.-Butylhydroxyanisol (BHA).

Die Polymerisationsinhibitoren des Bestandteils (d) sind im Unterschied zu den Molekulargewichtsreglern des Bestandteils (c) nicht zur H-Abstraktion in Allylstellung befähigt.

Sofern dennoch im Einzelfall ein Polymerisationsinhibitor gleichzeitig als ein zur H-Abstraktion in Allylstellung befähigter Molekulargewichtsregler aufgefasst werden kann, wird dieser für die Zwecke des vorliegenden Textes als Molekulargewichtsregler aufgefasst, insbesondere bei quantitativen Betrachtungen.

Die Kombination von einem oder mehreren Molekulargewichtsreglern des Bestandteil (c) und einem oder mehreren Polymerisationsinhibitoren des Bestandteils (d) in einer dualhärtenden, mehrkomponentigen erfindungsgemäßen dentalen Zusammensetzung führt dazu, insbesondere in den als bevorzugt gekennzeichneten Ausgestaltungen, dass nach dem Vermischen der zwei oder mehr Komponenten eine lange extraorale Verarbeitung der Zusammensetzung im vermischten Zustand ermöglicht wird und nach der intraoralen Applikation der Zusammensetzung im vermischten Zustand diese schnell aushärtet.

Die Anwesenheit eines oder mehrerer Polymerisationsinhibitoren des Bestandteils (d), vorzugsweise von BHT und/oder TEMPO, in einer erfindungsgemäßen dentalen Zusammensetzung, insbesondere in den oben genannten bevorzugten Mengen bzw. Mengenverhältnissen, ist deshalb vorteilhaft, weil die Lagerstabilität einer erfindungsgemäßen dentalen Zusammensetzung signifikant erhöht wird. Dabei wird die hier relevante Wirkung des oder der Molekulargewichtsregler (c) nicht beeinträchtigt.

Eine erfindungsgemäß bevorzugte dentale Zusammensetzung umfasst Bestandteil (d) in einer Menge 0,005 bis 0,100 Gew.-%, vorzugsweise von 0,005 bis 0,075 Gew.-%, bevorzugt von 0,010 bis 0,050 Gew.-%, jeweils bezogen auf die Gesamtmasse der dentalen Zusammensetzung.

Werden merklich höhere Mengen an Bestandteil (d) eingesetzt, so wird die Aushärtung der dentalen Zusammensetzung merklich verlangsamt.

In weiteren erfindungsgemäß bevorzugten dentalen Zusammensetzungen liegt das Verhältnis der Gesamtmasse des Bestandteils (c) zu der Gesamtmasse des Bestandteils (d) im Bereich von 1 : 5 bis 100 : 1, vorzugsweise im Bereich von 1 : 1 bis 20 : 1, bevorzugt im Bereich von 10 : 1 bis 20 : 1, weiter bevorzugt im Bereich von 11 : 1 bis 20 : 1, besonders bevorzugt im Bereich von 12 : 1 bis 18 : 1.

Zusammenfassend gewährleistet eine solche Kombination von Bestandteil (c) und (d) neben den oben bereits dargelegten Effekten, insbesondere in einer der als bevorzugt gekennzeichneten Ausgestaltungen, zudem
- eine sehr gute Lagerstabilität,
- bei Polymerisation der erfindungsgemäßen dentalen Zusammensetzung eine einstellbare / maßgeschneiderte Molekulargewichtsverteilung nach Aushärtung, und
- ein akzeptables Temperaturmaximum (Tₘₐₓ) bei der Polymerisation der dentalen Zusammensetzung im Mundraum.

### Bestandteil (e) - anorganische Füllstoffe

Die anorganischen Füllstoffe können allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder polymodale, beispielsweise eine bimodale Verteilung aufweisen.

Eine erfindungsgemäß bevorzugte dentale Zusammensetzung umfasst Bestandteil (e) in einer Menge von mindestens 10 Gew.-%, vorzugsweise von mindestens 20 Gew.-%, bevorzugt von mindestens 25 Gew.-%, bezogen auf die Gesamtmasse der dentalen Zusammensetzung.

Die Gesamtmenge an anorganischen Füllstoffen in einer erfindungsgemäßen dentalen Zusammensetzung beträgt vorzugsweise höchstens 95 Gew.-%, bevorzugt höchstens 90 Gew.-%, weiter bevorzugt höchstens 85 Gew.-%, bezogen auf die Gesamtmasse der Zusammensetzung.

Die Gesamtmenge an anorganischen Füllstoffen in einer erfindungsgemäßen dentalen Zusammensetzung beträgt vorzugsweise mindestens 25 Gew.-%, bevorzugt mindestens 30 Gew.-%, weiter bevorzugt mindestens 40 Gew.-%, bezogen auf die Gesamtmasse der Zusammensetzung.

Als anorganische Füllstoffe kommen kompakte Gläser und unterschiedliche Kieselsäuren in verschiedenen Größen und Zuständen (monodispers, polydispers) zum Einsatz.

Geeignete anorganische Bestandteile sind beispielsweise amorphe Materialien auf der Basis von Mischoxiden aus SiO₂ ZrO₂ und/oder TiO₂ mikrofeine Füllstoffe wie pyrogene Kieselsäure oder Fällungskieselsäure sowie Makro- oder Mini-Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zum besseren Einbau in die Polymermatrix können die Füllstoffe oberflächenmodifiziert sein. Beispielhaft genannt sei die Oberflächenbehandlung der Füllstoffe mit einem Silan. Als Haftvermittler eignet sich besonders das Methacryloxypropyltrimethoxysilan.

Zur Einstellung der Rheologie können erfindungsgemäße dentale Zusammensetzungen unterschiedliche Kieselsäuren, bevorzugt pyrogene Kieselsäuren, enthalten.

Daneben können verstärkend wirkende Materialien, wie Glasfasern, Polyamid- oder Kohlenstofffasern eingesetzt werden. Die dentale Zusammensetzung kann zusätzlich feinteilige Splitter oder Perlpolymerisate enthalten, wobei die Perlpolymerisate Homo- oder Copolymere organischer härtbarer Monomerer sein können.

In einer Vielzahl erfindungsgemäßer Zusammensetzungen wird vorzugsweise kein oder ein vergleichsweise geringer Anteil an organischen Füllstoffpartikeln eingesetzt, da manche dentale Anwendungen bestimmte, d.h. vergleichsweise hohe, mechanische Eigenschaften erfordern, die mit organischen Füllstoffpartikeln nicht erreichbar sind.

Sofern vorhanden, beträgt die Gesamtmenge an organischen Füllstoffpartikeln in einer erfindungsgemäßen dentalen Zusammensetzung vorzugsweise höchstens 40 Gew.-%, bevorzugt höchstens 35 Gew.-%, bezogen auf die Gesamtmasse der Zusammensetzung.

Sofern eine erfindungsgemäße dentale Zusammensetzung organische Füllstoffpartikel enthält, liegt deren Anteil vorzugsweise im Bereich von 5 bis 35 Gew.-%, bezogen auf die Gesamtmasse der Zusammensetzung.

Eine Vielzahl bevorzugter erfindungsgemäßer dentaler Zusammensetzungen umfasst keine organischen Füllstoffpartikel.

Die Gesamtmenge an anorganischen und organischen Füllstoffpartikeln in einer erfindungsgemäßen dentalen Zusammensetzung beträgt vorzugsweise höchstens 95 Gew.-%, bevorzugt höchstens 90 Gew.-%, weiter bevorzugt höchstens 85 Gew.-%, bezogen auf die Gesamtmasse der Zusammensetzung.

UV-Absorber, die beispielsweise durch ihre konjugierten Doppelbindungssytemen und aromatischen Ringe befähigt sind, UV Strahlung zu absorbieren, können gegebenenfalls Bestandteil einer erfindungsgemäßen dentalen Zusammensetzung sein. Beispiele an UV-Absorbern sind 2-Hydroxy-4-methoxybenzophenon, Salicylsäurephenylester oder 3-(2'-Hydroxy-5'-methylphenyl)-benzotriazol.

Da die Zähne möglichst naturgetreu wiederherzustellen sind, ist es notwendig, die erfindungsgemäßen dentalen Zusammensetzungen in den unterschiedlichsten Farbtönen bereitzustellen. Man benutzt zu diesem Zweck in der Regel anorganische Farbstoffe sowie organische Pigmente in sehr geringen Mengen.

Die jeweils vorstehend als bevorzugt oder besonders bevorzugt gekennzeichneten Ausgestaltungen einer erfindungsgemäßen Zusammensetzung gelten insbesondere auch in Kombination mit anderen als bevorzugt oder besonders bevorzugt gekennzeichneten Ausgestaltungen einer erfindungsgemäßen Zusammensetzung.

Entsprechend den vorstehenden Ausführungen umfasst eine besonders bevorzugte erfindungsgemäße dualhärtende, zweikomponentige dentale Zusammensetzung in Form zweier Pasten:
(a) ein oder mehrere photopolymerisierbare Monomere ausgewählt aus der Gruppe der Methacrylate,
(b) ein oder mehrere Photoinitiatoren, ausgewählt aus der Gruppe bestehend aus alpha-Diketonen, Benzoinalkylethern, Thioxanthonen, Benzophenonen, Acylphosphinoxiden, Acetophenonen, Ketalen, Titanocenen und Boraten, bevorzugt ausgewählt aus der Gruppe der alpha-Diketone, dabei bevorzugt Campherchinon,
(c) ein oder mehrere Molekulargewichtsregler ausgewählt aus der Gruppe bestehend aus α-Terpinen und γ-Terpinen,
(d) ein oder mehrere Polymerisationsinhibitoren zur Erhöhung der Lagerstabilität der Zusammensetzung, ausgewählt aus der Gruppe bestehend aus Hydrochinonmonomethylether (HQME), 2,6-Di-tert.butyl-4-methylphenol (BHT), tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) und Phenothiazin,
(e) ein oder mehrere anorganische Füllstöffe, vorzugsweise in einer Menge von mindestens 25 Gew.-%, bezogen auf das Gesamtgewicht der Zusammensetzung,
(f) ein oder mehrere Initiatoren für eine chemische Aushärtung bei Umgebungstemperatur, vorzugsweise Dibenzoylperoxid, bevorzugt Dibenzoylperoxid in Kombination mit N,N-Dimethyl-p-toluidin oder N,N-Dihydroxyethyl-p-toluidin,
wobei die Zusammensetzung vorzugsweise höchstens 5 Gew.-% an Alkylmonomethacrylaten und/oder vorzugsweise höchstens 40 Gew.-% an organischen Füllstoffpartikeln enthält.

Eine weiter besonders bevorzugte erfindungsgemäße zweikomponentige dentale Zusammensetzung in Form zweier Pasten umfasst:
(a) ein oder mehrere photopolymerisierbare Monomere ausgewählt aus der Gruppe der Methacrylate,
(b) ein oder mehrere Photoinitiatoren, ausgewählt aus der Gruppe bestehend aus alpha-Diketonen, vorzugsweise Campherchinon, bevorzugt Campherchinon in Kombination mit Ethyl-*p*-*N*,*N-*dimethylaminobenzoat,
(c) ein oder mehrere Molekulargewichtsregler ausgewählt aus der Gruppe bestehend aus α-Terpinen und γ-Terpinen,
(d) ein oder mehrere Polymerisationsinhibitoren zur Erhöhung der Lagerstabilität der Zusammensetzung, nämlich 2,6-Di-tert.butyl-4-methylphenol (BHT),
(e) ein oder mehrere anorganische Füllstoffe, in einer Menge von mindestens 30 Gew.-%, vorzugsweise in einer Menge von mindestens 40 Gew.-%, bezogen auf das Gesamtgewicht der Zusammensetzung,
(f) ein oder mehrere Initiatoren für eine chemische Aushärtung bei Umgebungstemperatur, vorzugsweise Dibenzoylperoxid in Kombination mit N,N-Dimethyl-p-toluidin oder N,N-Dihydroxyethyl-p-toluidin,
wobei das Verhältnis der Gesamtmasse des Bestandteils (c) zu der Gesamtmasse des Bestandteils (d) im Bereich von 10 : 1 bis 20 : 1 liegt, und
wobei die Zusammensetzung vorzugsweise höchstens 1 Gew.-% an Alkylmonomethacrylaten und/oder vorzugsweise höchstens 35 Gew.-% an organischen Füllstoffpartikeln enthält.

Die Erfindung betrifft ferner eine dentale Spritze, vorzugsweise eine Doppelspritze oder eine Doppelkammerkartusche, vorzugsweise mit einem statischen Mischrohr, enthaltend eine erfindungsgemäße dentale Zusammensetzung, vorzugsweise in einer der als bevorzugt gekennzeichneten Ausgestaltungen.

Die Erfindung betrifft ferner die Verwendung einer Verbindung mit zwei oder mehr Doppelbindungen, die durch Abstraktion eines Allyl-H-Atoms ein delokalisiertes Elektronensystem ausbilden kann, das sich über 5 oder mehr C-Atome der Verbindung erstreckt, bevorzugt eines Diens, das durch Abstraktion eines Allyl-H-Atoms ein delokalisiertes Elektronensystem ausbilden kann, das sich über 5 C-Atome erstreckt, vorzugsweise ausgewählt aus der Gruppe bestehend aus α-Terpinen und γ-Terpinen,
als Molekulargewichtsregler bei der Polymerisation von Monomeren ausgewählt aus der Gruppe bestehend aus Acrylaten und Methacrylaten, vorzugsweise aus der Gruppe der Methacrylate,
zur Herstellung einer dualhärtenden, mehrkomponentigen dentalen Zusammensetzung.

Die Erfindung betrifft ebenfalls ein Verfahren zur Herstellung einer erfindungsgemäßen dentalen Zusammensetzung, vorzugsweise in einer der als bevorzugt bzw. besonders bevorzugt gekennzeichneten Ausgestaltungen, mit folgenden Schritten:
- Herstellen eines mehrkomponentigen, vorzugsweise zweikomponentigen, Systems umfassend sämtliche der Bestandteile (a), (b), (c), (d), (e) und (f) sowie gegebenenfalls weitere Additive,
wobei die Initiatoren des Bestandteils (f) so auf separate Komponenten der dentalen Zusammensetzung verteilt sind, dass eine chemische Initiierung durch Vermischen der besagten Komponenten ausgelöst wird.

Ferner betrifft die Erfindung eine erfindungsgemäße dentale Zusammensetzung, vorzugsweise in einer der als bevorzugt bzw. besonders bevorzugt gekennzeichneten Ausgestaltungen, als dentales Füllungsmaterial, als fließfähiges Kompositmaterial (Flow-Material), als Kronenmaterial, als Brückenmaterial und/oder als Stumpfaufbaumaterial.

Ferner betrifft die Erfindung ein Verfahren zum Behandeln einer Zahnerkrankung, wobei eine erfindungsgemäße dentale Zusammensetzung, vorzugsweise in einer der als bevorzugt angegebenen Ausgestaltungen, als dentales Füllungsmaterial, als fließfähiges Kompositmaterial (Flow-Material), als Kronenmaterial, als Brückenmaterial und/oder als Stumpfaufbaumaterial eingesetzt wird.

Anhand der folgenden Beispiele wird die Erfindung weiter erläutert. Sofern nicht anders angegeben, beziehen sich alle Angaben auf das Gewicht. Es werden dabei branchenübliche Abkürzungen verwendet.

In den nachfolgenden Beispiele wurden die Basispaste (Basis) und die Katalysatorpaste (Kat.) im Gewichtsverhältnis 1 : 1 gemischt.

Beschreibung der Methode zur Bestimmung des Temperaturmaximums (Tₘₐₓ)

Die Wärmeentwicklung während der Polymerisation wurde mit einer relativen Messmethode bestimmt: ein Thermofühler Typ L (Fe-CuNi) der Fa. Reckmann wird in eine Halterung aus Polyethylen so eingebettet, dass 3 mm des Fühlers herausragen. Dieses freie Ende des Thermofühlers wird mittig in ein Polyethylenröhrchen von 3 mm Innendurchmesser und 5 mm Höhe platziert. Das Röhrchen wird mit Material randvoll gefüllt. Auf das gefüllte Röhrchen wird eine farblose klare Acetatfolie gelegt, so dass evtl. Materialüberschüsse verpresst werden. Um die Polymerisation auszulösen, wird eine dentale LED-Lampe mit 1000 mW/cm² Lichtleistung direkt auf die Acetatfolie aufgesetzt und für 10 sec eingeschaltet. Die aus der Temperaturentwicklung resultierende Spannungsänderung des Thermoelementes wird an einem xy-Schreiber L 250-2 der Fa. Linseis bei einer Empfindlichkeit von 2 mV aufgezeichnet.

Die Messung erfolgt in einem klimatisierten Raum bei 23°C und 50% relative Luftfeuchte unter gelbem Schutzlicht, um eine vorzeitige Polymerisation der Materialien zu vermeiden. Jede Einzelmessung wird erst dann gestartet (belichtet), wenn nach dem Auflegen der Acetatfolie wieder eine konstante Temperatur erreicht ist und der Messschreiber auf die Nulllinie justiert wurde. Gemessen wird der Maximalausschlag des Schreibers in Skalenteilen (0 - 100 Skt.). Der Klarheit halber sei angemerkt, dass die Skalenteile (Skt.) zwar mit der Temperatur korrelieren (d.h. innerhalb einer Messreihe entspricht ein höherer Skt.-Wert einer höheren Temperatur), jedoch zahlenmäßig nicht mit °C-Werten übereinstimmen. Die angegebenen Messwerte sind jeweils die Mittelwerte aus 3 Einzelmessungen.

**Tabelle 1: Ausführungsbeispiele mit γ-Terpinen**

| Bei den Mengenangaben handelt es sich um Gewichtsteile | | | | | | |
|---|---|---|---|---|---|---|
| | | Referenz | Exp. 1 | Exp. 2 | Exp.3 | Exp. 4 |
| Kat. | UDMA | 20,00 | 20,00 | 20,00 | 20,00 | 20,00 |
| | Ethoxyliertes Bisphenol-A-dimethacrylat | 40,00 | 40,00 | 40,00 | 40,00 | 40,00 |
| | BHT | 0,02 | 0,02 | 0,02 | 0,02 | 0,02 |
| | BPO | 0,25 | 0,25 | 0,25 | 0,25 | 0,25 |
| | Glaspulver | 35,00 | 35,00 | 35,00 | 35,00 | 35,00 |
| | Aerosil 1 | 2,00 | 2,00 | 2,00 | 2,00 | 2,00 |
| | Aerosil 2 | 3,50 | 3,50 | 3,50 | 3,50 | 3,50 |
| | | | | | | |
| Basis | UDMA | 20,00 | 20,00 | 20,00 | 20,00 | 20,00 |
| | Ethoxyliertes Bisphenol-A-dimethacrylat | 40,00 | 40,00 | 40,00 | 40,00 | 40,00 |
| | N,N-Di(hydroxyethyl)-p-toluidin | 2,00 | 2,00 | 2,00 | 2,00 | 2,00 |
| | Glaspulver | 35,00 | 35,00 | 35,00 | 35,00 | 35,00 |
| | Aerosil 1 | 2,00 | 2,00 | 2,00 | 2,00 | 2,00 |
| | Aerosil 2 | 3,50 | 3,50 | 3,50 | 3,50 | 3,50 |
| | Farbstoffe, Pigmente | 0,50 | 0,50 | 0,50 | 0,50 | 0,50 |
| | Campherchinon | 0,34 | 0,34 | 0,34 | 0,34 | 0,34 |
| | DABE | 0,51 | 0,51 | 0,51 | 0,51 | 0,51 |
| | BHT | 0,02 | 0,02 | 0,02 | 0,02 | 0,02 |
| | γ-Terpinen | | 0,20 | 0,50 | 1,00 | 2,00 |
| | | | | | | |
| | Tₘₐₓ [Skt] | 77 | 66 | 55 | 48 | 42 |

**Tabelle 1A-1: Umrechnung der Gewichtsanteile an BHT und γ-Terpinen in Gewichtsprozentangaben für die Zusammensetzungen gemäß Tabelle 1**

| | Referenz | Exp. 1 | Exp. 2 | Exp.3 | Exp. 4 |
|---|---|---|---|---|---|
| BHT (Gew.-%) | 0,02 | 0,02 | 0,02 | 0,02 | 0,02 |
| γ-Terpinen (Gew.-%) | 0,00 | 0,10 | 0,24 | 0,49 | 0,97 |

Die Angaben zu "BHT (Gew.-%)" und "γ-Terpinen (Gew.-%)" in Tabelle 1A-1 sind die Mengen an BHT bzw, γ-Terpinen in Gewichtsprozent (gerundet auf zwei Nachkommastellen), bezogen auf das Gesamtgewicht der jeweiligen Zusammensetzung gemäß Tabelle 1 nach Vermischen von Katalysatorpaste und Basispaste im Gewichtsverhältnis 1 : 1.

**Tabelle 1A-2: Abbinde- und Verarbeitungszeiten für die Zusammensetzungen gemäß Tabelle 1**

| | Referenz | Exp. 1 | Exp. 2 | Exp. 3 | Exp. 4 |
|---|---|---|---|---|---|
| Verarbeitungszeit | 50 sec | 80 sec | 96 sec | 120 sec | 150 sec |
| Abbindezeit | 53 sec | 57 sec | 60 sec | 64 sec | 70 sec |

Gemäß DIN EN ISO 4049 (wie oben spezifiziert) wurden die Verarbeitungszeiten bei 23°C und die Abbindezeiten bei 37°C für das Referenzbeispiel und die Beispiele Exp. 1 bis Exp. 4 gemäß Tabelle 1 bestimmt. Die jeweiligen Zeiten sind in Tabelle 1A-2 in Sekunden angegeben.

Es ist aus Tabelle 1A-2 erkennbar, dass die Abbindezeit von der Verarbeitungszeit mit steigendem Gehalt an γ-Terpinen zunehmend abweicht. Die ermittelten Daten für Verarbeitungszeit und Abbindezeit sind - wie oben bereits ausgeführt - in Fig. 1 graphisch dargestellt.

**Tabelle 1B: Nicht erfindungsgemäße Vergleichszusammensetzungen ohne γ-Terpinen mit variierendem Gehalt an BHT**

| | | | | | |
|---|---|---|---|---|---|
| BHT (Gew.-%) | 0,02 | 0,12 | 0,26 | 0,51 | 0,99 |
| Verarbeitungszeit | 50 sec | 65 sec | 81 sec | 96 sec | 118 sec |
| Abbindezeit | 53 sec | 68 sec | 85 sec | 100 sec | 125 sec |

Die Angaben zu "BHT (Gew.-%)" in Tabelle 1 B sind die Mengen an BHT in Gewichtsprozent (gerundet auf zwei Nachkommastellen), bezogen auf das Gesamtgewicht einer jeweiligen Zusammensetzung, deren Rezeptur mit der des Referenzbeispiels gemäß Tabelle 1 - nach Vermischen von Katalysatorpaste und Basispaste im Gewichtsverhältnis 1 : 1 - identisch ist, mit der Maßgabe, dass jeweils die oben in Tabelle 1B angegebenen Mengen an BHT verwendet wurden. Die Zusammensetzung der Katalysatorpaste blieb in jedem Falle identisch mit der aus dem Referenzbeispiel, lediglich die Zusammensetzung der Basispaste wurde durch Änderung des BHT-Anteils variiert.

Gemäß DIN EN ISO 4049 (wie oben spezifiziert) wurden die Verarbeitungszeiten bei 23°C und die Abbindezeiten bei 37°C für diese Vergleichszusammensetzungen bestimmt. Die jeweiligen Zeiten sind in Tabelle 1B in Sekunden angegeben. Die ermittelten Daten für Verarbeitungszeit und Abbindezeit sind - wie oben bereits ausgeführt - in Fig. 2 graphisch dargestellt.

**Tabelle 2: Weitere Ausführungsbeispiele mit γ-Terpinen**

| Bei den Mengenangaben handelt es sich um Gewichtsteile | | | | | |
|---|---|---|---|---|---|
| | | Exp. 5 | Exp. 6 | Exp. 7 | Exp. 8 |
| Kat. | UDMA | 35,00 | 18,07 | 15,00 | 15,00 |
| | Ethoxyliertes Bisphenol-A-dimethacrylat | | 39,15 | | |
| | Bis-GMA | | | 3,00 | 3,00 |
| | TEDMA | 15,00 | | | |
| | DODMA | | | 6,00 | 6,00 |
| | HEDMA | | | 6,00 | 6,00 |
| | PEG 300 | | 0,60 | | |
| | Weichmacher | | 0,50 | | |
| | BHT | 0,06 | 0,02 | 0,04 | 0,04 |

| | | Exp. 5 | Exp. 6 | Exp.7 | Exp. 8 |
|---|---|---|---|---|---|
| | BPO | 0,50 | 1,51 | 0,70 | |
| | 1,3-Dimethyl-5-phenylbarbitur-säure-Natriumsalz | | | | 1,20 |
| | Glaspulver | 30,00 | 35,13 | 66,50 | 66,00 |
| | Pyrogene Kieselsäure | 15,00 | 5,02 | 2,76 | 2,76 |
| | Titandioxid | 1,50 | | | |
| | | | | | |
| Basis | UDMA | | 18,30 | 14,00 | 14,00 |
| | Ethoxyliertes Bisphenol-A-dimethacrylat | | 37,89 | | |
| | TEDMA | 20,00 | | | |
| | DODMA | | | 6,00 | 6,00 |
| | HEDMA | | | 5,50 | 5,50 |
| | PEG 300 | | 0,60 | | |
| | Weichmacher | | 0,52 | | |
| | Bis-GMA | 35,00 | | 3,00 | 3,00 |
| | Glaspulver | 27,00 | 35,24 | 67,50 | 67,50 |
| | Pyrogene Kieselsäure | 14,00 | 4,38 | 3,00 | 3,00 |
| | Titandioxid | 1,50 | | | |
| | Farbstoffe, Pigmente | | 0,34 | 0,01 | 0,01 |
| | Campherchinon | 0,01 | 0,07 | 0,08 | 0,04 |
| | Cu(II)-acetylacetonat | | | | 0,25 |
| | Benzyltributylammoniumchlorid | | | | 0,25 |
| | DABE | | 0,10 | 0,11 | 0,1 |
| | N,N-Di(hydroxyethyl)-p-toluidin | 0,41 | 2,24 | 0,45 | |
| | γ-Terpinen | 0,70 | 0,34 | 0,35 | 0,35 |
| | | | | | |
| | Tₘₐₓ [Skt] | 48 | 46 | 49 | 43 |

Gemäß DIN EN ISO 4049 (wie oben spezifiziert) wurden die Verarbeitungszeiten bei 23°C und die Abbindezeiten bei 37°C für die Beispiele Exp. 5 bis Exp. 8 bestimmt. Die jeweiligen Zeiten sind in Sekunden angegeben.

**Tabelle 3: Verarbeitungs- und Abbindezeiten der Beispiele Exp. 5 bis Exp. 8**

| | Exp.5 | Exp.6 | Exp.7 | Exp.8 |
|---|---|---|---|---|
| Verarbeitungszeit | 90 sec | 105 sec | 80 sec | 110 sec |
| Abbindezeit | 57 sec | 62 sec | 57 sec | 64 sec |

## Patentansprüche

1. Dualhärtende, mehrkomponentige dentale Zusammensetzung umfassend:
(a) ein oder mehrere photopolymerisierbare Monomere ausgewählt aus der Gruppe bestehend aus Acrylaten und Methacrylaten,
(b) ein oder mehrere Photoinitiatoren, ausgewählt aus der Gruppe bestehend aus alpha-Diketonen, Benzoinalkylethern, Thioxanthonen, Benzophenonen, Acylphosphinoxiden, Acetophenonen, Ketalen, Titanocenen, Boraten und sensibilisierenden Farbstoffen,
(c) ein oder mehrere Molekulargewichtsregler ausgewählt aus der Gruppe bestehend aus Verbindungen, die mit einem Radikal eines Monomers des Bestandteils (a) umsetzbar sind, wobei die Umsetzung unter Abstraktion eines H-Radikals aus dem Molekulargewichtsregler in Allylposition erfolgt,
und
wobei ein oder mehrere der Molekulargewichtsregler des Bestandteils (c) Verbindungen mit zwei oder mehr Doppelbindungen sind, die durch Abstraktion eines Allyl-H-Atoms ein delokalisiertes Elektronensystem ausbilden können, das sich über 5 oder mehr C-Atome erstreckt,
und ausgewählt sind aus der Gruppe der Monoterpene α-Terpinen, β-Terpinen, γ-Terpinen, α-Phellandren, β-Phellandren und Terpinolen, oder ausgewählt sind aus der Gruppe bestehend aus Linolsäure, Linolensäure und sonstigen substituierten oder nicht substituierten Cyctohexadienen
(d) ein oder mehrere Polymerisationsinhibitoren zur Erhöhung der Lagerstabilität der Zusammensetzung, ausgewählt aus der Gruppe bestehend aus Hydrochinonmonomethylether (HQME), 2,6-Di-tert.butyl-4-methylphenol (BHT), tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) und Phenothiazin,
(e) ein oder mehrere anorganische Füllstoffe,
(f) ein oder mehrere Initiatoren für eine chemische Aushärtung bei Umgebungstemperatur,
sowie gegebenenfalls ein oder mehrere weitere Additive.

2. Dentale Zusammensetzung nach Anspruch 1, wobei die dentale Zusammensetzung zweikomponentig ist.

3. Dentale Zusammensetzung nach einem der vorangehenden Ansprüche, wobei die dentale Zusammensetzung zweikomponentig ist und in Form einer Basispaste und einer Katalysatorpaste vorliegt.

4. Dentale Zusammensetzung nach einem der vorangehenden Ansprüche, insbesondere nach Anspruch 1, wobei Bestandteil (f) umfasst:
(f-a)
- ein oder mehrere Initiatoren, die ausgewählt sind aus der Gruppe bestehend aus anorganischen Peroxiden, organischen Peroxiden, anorganischen Hydroperoxiden, organischen Hydroperoxiden, Barbitursäurederivaten, Malonylsulfamiden, Protonensäuren, Lewis- oder Broensted-Säuren, solche Säuren freisetzenden Verbindungen und Carbeniumionen-Donatoren;
- ein oder mehrere Co-Initiatoren ausgewählt aus der Gruppe bestehend aus tertiären Aminen, Schwermetallverbindungen, Verbindungen mit ionogen gebundenen Halogenen oder Pseudohalogenen und schwachen Broenstedt-Säuren,
und/oder
(f-b) ein Redoxinitiatorsystem umfassend
(f-b1) Barbitursäure, Thiobarbitursäure, ein Barbitursäure- und/oder ein Thiobarbitursäurederivat,
(f-b2) gegebenenfalls eine Peroxodisulfat- und/oder eine Peroxodiphosphatverbindung,
(f-b3) eine Kupferverbindung,
(f-b4) eine Verbindung mit einem ionogen vorliegenden Halogenatom,
wobei die Initiatoren des Bestandteils (f) so auf separate Komponenten der dentalen Zusammensetzung verteilt sind, dass eine chemische Initiierung durch Vermischen der besagten Komponenten ausgelöst wird.

5. Dentale Zusammensetzung nach Anspruch 3 oder 4, wobei die Katalysatorpaste einen oder mehrere Initiatoren des Bestandteils (f-a) enthält und die Basispaste einen oder mehrere Co-Initiatoren des Bestandteils (f-a) enthält.

6. Dentale Zusammensetzung nach einem der vorangehenden Ansprüche, **dadurch gekennzeichnet, dass** ein oder mehrere der Molekulargewichtsregler des Bestandteils (c) ausgewählt sind aus der Gruppe bestehend aus α-Terpinen und γ-Terpinen.

7. Dentale Zusammensetzung nach einem der vorangehenden Ansprüche, umfassend
Bestandteil (a) in einer Menge von 19 bis 70 Gew.-%,
Bestandteil (c) in einer Menge von 0,05 bis 5,00 Gew.-% und
Bestandteil (e) in einer Menge von 25 bis 80 Gew.-%,
jeweils bezogen auf die Gesamtmasse der dentalen Zusammensetzung.

8. Dentale Zusammensetzung nach einem der vorangehenden Ansprüche, umfassend
Bestandteil (a) in einer Menge von 19 bis 70 Gew.-%,
Bestandteil (b) in einer Menge von 0,05 bis 1,00 Gew.-%,
Bestandteil (c) in einer Menge von 0,05 bis 5,00 Gew.-%,
Bestandteil (e) in einer Menge von 25 bis 80 Gew.-%und
Bestandteil (f) in einer Menge von 0,2 bis 2 Gew.-%,
jeweils bezogen auf die Gesamtmasse der dentalen Zusammensetzung.

9. Dentale Zusammensetzung nach einem der vorangehenden Ansprüche, wobei die Zusammensetzung neben einem oder mehreren Photoinitiatoren des Bestandteils (b) zusätzlich einen oder mehrere von Bestandteil (b) verschiedene Initiatoren des Bestandteils (f) für eine chemische Aushärtung bei Umgebungstemperatur enthält.

10. Dentale Zusammensetzung nach einem der vorangehenden Ansprüche, **dadurch gekennzeichnet, dass** Bestandteil (a) ein oder mehrere Dimethacrylat-Monomere enthält gewählt aus der Gruppe bestehend aus Ethylenglykoldimethacrylat (EGDMA), 1,6-Hexandioldimethacrylat (HEDMA), Triethylenglykoldimethacrylat (TEGDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat, Bisphenol-A-Glycidyl-Methacrylat (Bis-GMA) und Dimethacrylaten des Dihydroxymethyltricyclo[5.2.1.0^{2,6}]decans.

11. Dentale Zusammensetzung nach einem der vorangehenden Ansprüche, **dadurch gekennzeichnet, dass**
diese Bestandteil (d) in einer Menge von 0,005 bis 0,100 Gew.-% umfasst bezogen auf die Gesamtmasse der dentalen Zusammensetzung,
und/oder
das Verhältnis der Gesamtmasse des Bestandteils (c) zu der Gesamtmasse des Bestandteils (d) im Bereich von 1 : 5 bis 100 : 1 liegt.

12. Dentale Zusammensetzung nach einem der vorangehenden Ansprüche als dentales Füllungsmaterial, als fließfähiges Kompositmaterial, als Kronenmaterial, als Brückenmaterial und/oder als Stumpfaufbaumaterial.

13. Dentale Spritze enthaltend eine dentale Zusammensetzung nach einem der vorangehenden Ansprüche.

14. Verfahren zur Herstellung einer dentalen Zusammensetzung nach einem der vorangehenden Ansprüche, mit folgenden Schritten:
- Herstellen eines mehrkomponentigen Systems umfassend sämtliche der Bestandteile (a), (b), (c), (d), (e) und (f) sowie gegebenenfalls weitere Additive,
wobei die Initiatoren des Bestandteils (f) so auf separate Komponenten der dentalen Zusammensetzung verteilt sind, dass eine chemische Initiierung durch Vermischen der besagten Komponenten ausgelöst wird.

15. Verwendung einer Verbindung mit zwei oder mehr Doppelbindungen, die durch Abstraktion eines Allyl-H-Atoms ein delokalisiertes Elektronensystem ausbilden kann, das sich über 5 oder mehr C-Atome der Verbindung erstreckt als Molekulargewichtsregler bei der Polymerisation von Monomeren ausgewählt aus der Gruppe bestehend aus Acrylaten und Methacrylaten
zur Herstellung einer dualhärtenden, mehrkomponentigen dentalen Zusammensetzung nach einem der Ansprüche 1 bis 12.

## Claims

1. A dual-curing, multi-component dental composition comprising:
(a) one or more photopolymerisable monomers selected from the group consisting of acrylates and methacrylates,
(b) one or more photoinitiators selected from the group consisting of alpha-diketones, benzoin alkyl ethers, thioxanthones, benzophenones, acyl phosphine oxides, acetophenones, ketals, titanocenes, borates and sensitising dyes,
(c) one or more molecular weight regulators selected from the group consisting of compounds that can be reacted with a radical of a monomer of component (a), wherein the reaction takes place with abstraction of an H radical from the molecular weight regulator in the allyl position,
and
wherein one or more of the molecular weight regulators of component (c) are compounds with two or more double bonds, which, through abstraction of an allyl H atom, can form a delocalised electron system extending across 5 or more C atoms,
and are selected from the group of the monoterpenes α-terpinene, β-terpinene, γ-terpinene, α-phellandrene, β-phellandrene and terpinolene or are selected from the group consisting of linoleic acid, linolenic acid and other substituted or non-substituted cyclohexadienes,
(d) one or more polymerisation inhibitors for increasing the storage stability of the composition, selected from the group consisting of hydroquinone monomethyl ether (HQME), 2,6-di-tert.-butyl-4-methylphenol (BHT), tert.-butylhydroxyanisole (BHA), 2,2-diphenyl-1-picrylhydrazyl radicals, galvinoxyl radicals, triphenylmethyl radicals, 2,3,6,6-tetramethylpiperidinyl-1-oxyl free radical (TEMPO) and phenothiazine,
(e) one or more inorganic fillers,
(f) one or more initiators for chemical curing at ambient temperature
and optionally one or more further additives.

2. The dental composition according to claim 1, wherein the dental composition is a two-component composition.

3. The dental composition according to one of the preceding claims, wherein the dental composition is a two-component composition and is present in the form of a base paste and a catalyst paste.

4. The dental composition according to one of the preceding claims, in particular according to claim 1, wherein component (f) comprises:
(f-a)
- one or more initiators, which are selected from the group consisting of inorganic peroxides, organic peroxides, inorganic hydroperoxides, organic hydroperoxides, barbituric acid derivatives, malonyl sulfamides, protonic acids, Lewis or Bronsted acids, compounds releasing such acids and carbenium ion donors;
- one or more co-initiators selected from the group consisting of tertiary amines, heavy metal compounds, compounds with ionically bound halogens or pseudohalogens and weak Bronsted acids
and/or
(f-b) a redox initiator system, comprising
(f-b1) barbituric acid, thiobarbituric acid, a barbituric acid derivative and/or a thiobarbituric acid derivative,
(f-b2) optionally a peroxodisulfate and/or a peroxodiphosphate compound,
(f-b3) a copper compound,
(f-b4) a compound with an ionically present halogen atom,
wherein the initiators of component (f) are distributed over separate components of the dental composition in such a way that a chemical initiation is triggered through the mixing of said components.

5. The dental composition according to claim 3 or 4, wherein the catalyst paste contains one or more initiators of component (f-a) and the base paste contains one or more co-initiators of component (f-a).

6. The dental composition according to one of the preceding claims, **characterised in that** one or more of the molecular weight regulators of component (c) are selected from the group consisting of α-terpinene and γ-terpinene.

7. The dental composition according to one of the preceding claims, comprising
component (a) in a quantity of 19 to 70 wt.%,
component (c) in a quantity of 0.05 to 5.00 wt.% and
component (e) in a quantity of 25 to 80 wt.%,
based in each case on the total mass of the dental composition.

8. The dental composition according to one of the preceding claims, comprising
component (a) in a quantity of 19 to 70 wt.%,
component (b) in a quantity of 0.05 to 1.00 wt.%,
component (c) in a quantity of 0.05 to 5.00 wt.%,
component (e) in a quantity of 25 to 80 wt.% and
component (f) in a quantity of 0.2 to 2 wt.%,
based in each case on the total mass of the dental composition.

9. The dental composition according to one of the preceding claims, wherein, as well as one or more photoinitiators of component (b), the composition additionally contains one or more initiators of component (f) that are different from component (b) for chemical curing at ambient temperature.

10. The dental composition according to one of the preceding claims, **characterised in that** component (a) contains one or more dimethacrylate monomers selected from the group consisting of ethylene glycol dimethacrylate (EGDMA), 1,6-hexanediol dimethacrylate (HEDMA), triethylene glycol dimethacrylate (TEGDMA), 1,12-dodecanediol dimethacrylate (DODMA), ethoxylated bisphenol A dimethacrylate, polyethylene glycol dimethacrylate (PEGDMA), 7,7,9-trimethyl-4,13-dioxo-5,12-diazahexadecane 1,16-dioxydimethacrylate (UDMA), butanediol dimethacrylate, tetraethylene glycol dimethacrylate, neopentyl glycol dimethacrylate, 2-hydroxypropyl 1,3-dimethacrylate, 3-hydroxypropyl 1,2-dimethacrylate, pentaerythritol dimethacrylate, glycerol dimethacrylate, bisphenol A glycidyl methacrylate (bis-GMA) and dimethacrylates of dihydroxymethyltricyclo[5.2.1.0^{2.6}]decane.

11. The dental composition according to one of the preceding claims, **characterised in that**
said composition comprises component (d) in a quantity of 0.005 to 0.100 wt.%, based on the total mass of the dental composition,
and/or
the ratio of the total mass of component (c) to the total mass of component (d) is in the range 1:5 to 100:1.

12. The dental composition according to one of the preceding claims as a dental filling material, as a flowable composite material, as a crown material, as a bridge material and/or as a stump build-up material.

13. A dental syringe containing a dental composition according to one of the preceding claims.

14. A method for producing a dental composition according to one of the preceding claims, with the following steps:
- production of a multi-component system comprising all of the components (a), (b), (c), (d), (e) and (f) and optionally further additives,
wherein the initiators of component (f) are distributed over separate components of the dental composition in such a way that a chemical initiation is triggered through the mixing of said components.

15. Use of a compound having two or more double bonds, which compound, through abstraction of an allyl H atom, can form a delocalised electron system extending across 5 or more C atoms of the compound, as a molecular weight regulator in the polymerisation of monomers selected from the group consisting of acrylates and methacrylates
for the production of a dual-curing, multi-component dental composition according to one of claims 1 to 12.

## Revendications

1. Composition dentaire multicomposant à durcissement double comprenant :
(a) un ou plusieurs monomères photopolymérisables choisis dans le groupe constitué d'acrylates et de méthacrylates,
(b) un ou plusieurs photo-initiateurs choisis dans le groupe constitué d'alpha-dicétones, d'alkyléthers de benzoïne, de thioxanthones, de benzophénones, d'oxydes d'acylphosphine, d'acétophénones, de cétals, de titanocènes, de borates et de colorants sensibilisants,
(c) un ou plusieurs régulateurs de poids moléculaire choisis dans le groupe constitué de composés qui peuvent réagir avec un radical d'un monomère du constituant (a), la réaction s'effectuant par abstraction d'un radical H du régulateur de poids moléculaire en position allyle,
et
un ou plusieurs des régulateurs de poids moléculaire du constituant (c) étant des composés comportant deux doubles liaisons ou plus, qui peuvent former par abstraction d'un atome de H d'allyle, un système d'électrons délocalisés qui s'étend sur 5 atomes de C ou plus,
et sont choisis dans le groupe des monoterpènes α-terpinène, β-terpinène, γ-terpinène, α-phéllandrène, β-phéllandrène et terpinols ou sont choisis dans le groupe constitué d'acide linoléique, d'acide linolénique et autres cyclohexadiènes substitués ou non substitués,
(d) un ou plusieurs inhibiteurs de polymérisation pour augmenter la stabilité au stockage de la composition, choisis dans le groupe constitué de monométhyléther d'hydroquinone (HQME), de 2,6-di-tert-butyl-4-méthylphénol (BHT), de tert-butylhydroxyanisol (BHA), de radicaux de 2,2-diphényl-1-picrylhydrazyle, de radicaux de galvinoxyle, de radicaux de triphénylméthyle, de radical de 2,3,6,6-tétraméthylpipéridinyl-1-oxyle (TEMPO) et de phénothiazine,
(e) un ou plusieurs agents de charge inorganiques,
(f) un ou plusieurs initiateurs pour un durcissement chimique à température ambiante,
et éventuellement un ou plusieurs autres additifs.

2. Composition dentaire selon la revendication 1, la composition dentaire étant à deux composants.

3. Composition dentaire selon l'une des revendications précédentes, la composition dentaire étant à deux composants et se présentant sous la forme d'une pâte de base et d'une pâte de catalyseur.

4. Composition dentaire selon l'une des revendications précédentes, en particulier selon la revendication 1, le constituant (f) comprenant :
(f-a)
- un ou plusieurs initiateurs qui sont choisis dans le groupe constitué de peroxydes inorganiques, de peroxydes organiques, d'hydroperoxydes inorganiques, d'hydroperoxydes organiques, de dérivés d'acide barbiturique, de malonylsulfamides, d'acides protoniques, d'acides de Lewis ou de Broensted, ces composés libérant des acides et des donneurs d'ions carbénium ;
- un ou plusieurs co-initiateurs choisis dans le groupe constitué
d'amines tertiaires, de composés de métaux lourds, de composés comportant des halogènes ou pseudo-halogènes liés de façon ionogène et des acides de Broenstedt faibles,
et/ou
(f-b) un système d'initiateurs redox comprenant
(f-b1) un acide barbiturique, un acide thiobarbiturique, un dérivé d'acide barbiturique et/ou un dérivé d'acide thiobarbiturique,
(f-b2) éventuellement un composé de peroxodisulfate et/ou un composé de peroxodiphosphate,
(f-b3) un composé de cuivre,
(f-b4) un composé comportant un halogénoatome se présentant sous forme ionogène
les initiateurs du constituant (f) étant répartis sur des composants séparés de la composition dentaire, de telle sorte qu'une initiation chimique soit déclenchée par mélange desdits composants.

5. Composition dentaire selon la revendication 3 ou 4, la pâte de catalyseur contenant un ou plusieurs initiateurs du constituant (f-a) et la pâte de base contenant un ou plusieurs co-initiateurs du constituant (f-a).

6. Composition dentaire selon l'une des revendications précédentes, **caractérisée en ce qu'**un ou plusieurs régulateurs de poids moléculaire du constituant (c) sont choisis dans le groupe constitué de α-terpines et de γ-terpines.

7. Composition dentaire selon l'une des revendications précédentes, comprenant
le constituant (a) en une quantité de 19 à 70 % en poids,
le constituant (c) en une quantité de 0,05 à 5,00 % en poids et
le constituant (e) en une quantité de 25 à 80 % en poids,
respectivement par rapport à la masse totale de la composition dentaire.

8. Composition dentaire selon l'une des revendications précédente, comprenant
le constituant (a) en une quantité de 19 à 70 % en poids,
le constituant (b) en une quantité de 0,05 à 1,00 % en poids,
le constituant (c) en une quantité de 0,05 à 5,00 % en poids,
le constituant (e) en une quantité de 25 à 80 % en poids et
le constituant (f) en une quantité de 0,2 à 2 % en poids,
respectivement par rapport à la masse totale de la composition dentaire.

9. Composition dentaire selon l'une des revendications précédentes, la composition contenant, outre un ou plusieurs photo-initiateurs du constituant (b) en outre un ou plusieurs initiateurs du constituant (f) différents du constituant (b) pour un durcissement chimique à température ambiante.

10. Composition dentaire selon l'une des revendications précédentes, **caractérisée en ce que** le constituant (a) contient un ou plusieurs monomères de diméthacrylate choisis dans le groupe constitué de diméthacrylate d'éthylène glycol (EGDMA), de 1,6-hexanedioldiméthacrylate (HEDMA), de diméthacrylate de triéthylène glycol (TEGDMA), de diméthacrylate de 1,12-dodécanediol (DODMA), de diméthacrylate de bisphénol A éthoxylé, de diméthacrylte de polyéthylène glycol (PEGDMA), de 7,7,9-triméthyl-4,13-dioxo-5,12-diazahexadécane-1,16-dioxydiméthacrylate (UDMA), de diméthacrylate de butanediol, de diméthacrylate de tétraéthylène glycol, de diméthacrylate de néopentylglycol, de 2-hydroxypropyl-1,3-diméthacrylate, de 3-hydroxypropyl-1,2-diméthacrylate, de penta-érythritol-diméthacrylate, de diméthacrylate de glycérine, de méthacrylate de bisphénol-A-glycidyle (Bis-GMA) et de diméthacrylates de dihydroxyméthyltricyclo[5.2.1.0^{2,6}]décane.

11. Composition dentaire selon l'une des revendications précédentes, **caractérisée en ce que**
ce constituant (d) est en une quantité de 0,005 à 0,100 % en poids par rapport à la masse totale de la composition dentaire
et/ou
le rapport de la masse totale du constituant (c) à la masse totale du constituant (d) se situe dans la plage de 1 : 5 à 100 : 1.

12. Composition dentaire selon l'une des revendications précédentes comme agent de comblement dentaire, comme matériau composite fluide, comme matériau de couronne, comme matériau de bridge et/ou comme matériau de constitution de moignon.

13. Seringue dentaire contenant une composition dentaire selon l'une des revendications précédentes.

14. Procédé de production d'une composition dentaire selon l'une des revendications précédentes, comprenant les étapes suivantes :
- production d'un système à plusieurs composants comprenant tous les constituants (a), (b), (c), (d), (e) et (f) et éventuellement d'autres additifs,
les initiateurs du constituant (f) étant répartis sur des composants séparés de la composition dentaire de telle sorte qu'une initiation chimique soit déclenchée par mélange desdits composants.

15. Utilisation d'un composé comportant deux doubles liaisons ou plus, qui peut former, par abstraction d'un atome de H d'allyle, un système d'électrons délocalisé qui s'étend sur 5 atomes de C ou plus du composé comme régulateur de poids moléculaire lors de la polymérisation de monomères choisis dans le groupe constitué d'acrylates et de méthacrylates,
pour la production d'une composition dentaire multicomposant à durcissement double selon l'une des revendications 1 à 12.
